# EUROPEAN PATENT APPLICATION

(11) **EP 2 684 962 A1**
(43) Date of publication of application: **15.01.2014**
(21) Application number: 12305828.1
(22) Date of filing: 10.07.2012
(51) Int. Cl.: C12N 15/86, C12N 15/11

(54) **Vector for the selective silencing of a gene in astrocytes**

(71) Applicant: COMMISSARIAT A L'ENERGIE ATOMIQUE ET AUX ENERGIES ALTERNATIVES, 75015 Paris (FR)
(72) Inventor: Deglon, Nicole, 1521 Curtilles (CH)
(74) Representative: Vial, Lionel

(57) **Abstract**

The present invention relates to a viral vector for silencing a gene specifically in astrocytes comprising:
- an astrocyte-specific viral envelope protein,
- a first nucleic acid sequence encoding a transcription activator and at least one target sequence of a neuron-specific miR under the control of an astrocyte-specific promoter, and
- a second nucleic acid sequence encoding a RNA for silencing the gene under the control of a promoter inducible by the transcription activator.

## Description

### Technical field

The present invention relates to a vector for the selective silencing of gene in astrocytes and uses thereof.

### Technical background

Increasing evidences show the importance of astrocytes in the development of neurodegenerative pathologies (Bradford *et al.,* 2009; Bradford *et al.,* 2010; Faideau *et al.,* 2010; Powers *et al.,* 2007; Shin *et al.,* 2005). In Huntington's disease (HD), neurodegeneration is strongly influenced by the toxicity linked to the production of mutant huntingtin (mHtt) in both neurons and astrocytes (Lobsiger and Cleveland, 2007). The latter have numerous roles in supporting neurons such as maintaining the blood-brain barrier, regulating the blood flow, the homeostasis of ions and water, the synaptic transmission and the energy metabolism (Haydon and Carmignoto, 2006; Maragakis and Rothstein, 2006; Slezak and Pfrieger, 2003; Takano *et al.,* 2006; Volterra and Meldolesi, 2005; Voutsinos-Porche *et al.,* 2003). Nevertheless, the concrete role of astrocytes into neuronal dysfunction and death in HD pathology remains to be determined.

To overexpress a gene specifically in astrocytes, most strategies rely on constitutive or conditional transgene expression in astroglial cells (Hirrlinger *et al.,* 2006; Mori *et al.,* 2006; Slezak *et al.,* 2007). Among these, one may cite tamoxifen (TAM) inducible CreERT2/loxP system or a constitutive expression of the transgene from an astrocytic promoter such as the GFAP-HD mice model (Bradford *et al.,* 2009). In these models, the transgene is expressed in astrocytes of almost all brain regions. However, in GFAP-CreERT2-transgenic mice, recombination in neurons is also occurring, since the GFAP promoter is active in embryonic radial glia that possesses a substantial neurogenic potential. In addition, GFAP-driven transgene expression in striatum, the main affected brain area in HD, is very low under physiological conditions (Hirrlinger *et al.,* 2006). Differences in recombination efficiency between transgenic lines complicate the analysis and it is still not always possible to target specific cell subpopulations through transgenesis. Hence, studying the specific role of astrocytes in HD requires the development of tools specifically targeting this particular cell population in one targeted brain structure.

The development of highly efficient viral vectors for gene transfer in the CNS is providing new systems for localized and controlled gene expression in a subset of cell population (Jakobsson and Lundberg, 2006; Wong *et al.,* 2006). Pseudotyping lentiviral vectors (LVs) with the glycoprotein of the vesicular stomatitis virus (VSV-G) confer a high neurotropism when combined with the ubiquitous promoter of the phosphoglycerate kinase 1 (PGK) (Deglon *et al.,* 2000). In contrast, LVs pseudotyped with the glycoprotein of the Mokola virus (MOK-G) transduce both neurons and glial cells (Cannon *et al.,* 2010; Desmaris *et al.,* 2001; Pertusa *et al.,* 2008; Watson *et al.,* 2002). Furthermore, it has been shown that addition of a micro-RNA target (miRT) restricts transgene expression to cell subpopulations (Brown *et al.,* 2007a; Brown *et al.,* 2007b; Brown *et al.,* 2006). Combining MOK-G pseudotyping with miR124T detargeting is associated with an astrocytic targeting of LVs (Colin *et al.,* 2009). Colin and colleagues have used miR124, which is highly and specifically expressed in neurons (Deo *et al.,* 2006; Lagos-Quintana *et al.,* 2002; Mishima *et al.,* 2007; Smimova *et al.,* 2005) to overexpress genes of interest into striatal astrocytes *in vivo.* However, this system is not suitable for silencing a ubiquitous gene because of the maturation of a small interfering RNA (siRNA) by dicer will cleaved the miRT located at the 3'-end of the mRNA.

### Description of the invention

The inventors have now shown that it was possible to overcome this issue and to selectively silence a ubiquitous gene in astrocytes, by combining four methodologies: i) tissue-specific promoters, ii) tetracycline-regulated expression system, iii) miR embedded siRNA expression, and iv) LV pseudotyping.

Thus the present invention relates to a viral vector for silencing a gene specifically in astrocytes comprising:
- an astrocyte-specific viral envelope protein,
- a first nucleic acid sequence encoding a transcription activator and at least one target sequence of a neuron-specific miR under the control of an astrocyte-specific promoter, and
- a second nucleic acid sequence encoding a RNA for silencing the gene under the control of a promoter inducible by the transcription activator.

As intended herein, "astrocytes" are a sub-type of glial cells, i.e. non neuronal cells, in the central nervous system. They are also known as astrocytic glial cells. Astrocytes are star-shaped and usually express the S100β marker and the Glial Fibrillary Acidic Protein (GFAP).

As intended herein, an "astrocyte-specific viral envelope protein" relates to an envelope protein, in particular an envelope glycoprotein, from a virus which promotes the binding of a vector carrying it, i.e. a vector which is pseudotyped by the envelope protein, to astrocytes. More preferably, an astrocyte-specific viral envelope protein according to the invention promotes a preferential binding of the vector carrying it to astrocytes over at least one cell type other than astrocytes, in particular over neurons.

Preferably, the astrocyte-specific viral envelope protein according to the invention is selected from the group consisting of Mokola virus G protein (G-MOK), rabies G protein, lymphocytic choriomeningitis virus envelope (LCMV), and moloney murine leukemia virus envelope (MuMLV).

More preferably, the astrocyte-specific viral envelope protein according to the invention is Mokola virus G protein (G-MOK).

The "transcription activator" according to the invention is a protein compound which upon binding to a particular locus on the vector will activate the promoter inducible by the transcription activator, which will enable or increase the transcription of the sequence(s) which are controlled by the promoter. As intended herein, the locus may be located next to the promoter or be comprised in the promoter or may be located at distance from the promoter. Numerous transcription activator/ promoter inducible by the transcription activator couples according to the invention are known in art.

Preferably, the transcription activator/ promoter inducible by the transcription activator according to the invention is selected from the group consisting of a tetracycline transactivator/tetracycline response element (TRE) and a tetracycline transrepressor/tetracycline response element (TRE).

More preferably, the transcription activator according to the invention is the tetracycline transactivator (tTA) and the promoter inducible by the transcription activator is the tetracycline response element (TRE). The tetracycline transactivator is preferably encoded by a sequence comprising or consisting of SEQ ID NO: 1 and the tetracycline response element preferably comprises 7 copies of the tetO operator along with the CMV promoter and is more preferably represented by a sequence comprising or consisting of SEQ ID NO: 2.

As intended herein an "astrocyte-specific promoter" according to the invention relates to a promoter which is constitutively active in astrocytes. More preferably, an astrocyte-specific promoter according to the invention promotes a preferential transcription of sequences within astrocytes over at least one cell type other than astrocytes, in particular over neurons.

Preferably, the astrocyte-specific promoter according to the invention is selected from the group consisting of the promoter of the glutamine synthase (GS) gene, the promoter the human excitatory amino acids transporter 1 (hEAAT1) and the cytomegalovirus (CMV) promoter.

More preferably, the astrocyte-specific promoter according to the invention is the promoter of the glutamine synthase (GS) gene, more preferably the promoter of rat GS gene. The promoter of rat GS gene is preferably represented by a sequence comprising SEQ ID NO: 3.

As intended herein a "neuron-specific miR" according to the invention relates to a microRNA which is present in neurons, in particular in human neurons. More preferably, a neuron-specific miR according to the invention is essentially not expressed in astrocytes. The target sequence of a miR preferably comprises or consists of a sequence complementary to the sequence of the miR.

Preferably, the target sequence of the neuron-specific miR according to the invention is selected from the group consisting of a target sequence of miR124 in particular hsa-miR124, miR128, in particular hsa-miR128, and miR10, in particular hsa-miR10. As will be clear to one of skill in the art "hsa" stands for *Homo sapiens.*

More preferably, the target sequence of the neuron-specific miR according to the invention is a target sequence of miR124 (miR124T), wherein miR124 most preferably relates to hsa-miR124-3p (SEQ ID NO: 14). miR124T is preferably encoded by a sequence comprising or consisting of SEQ ID NO: 4.

As will be clear to one of skill in the art, the viral vector of the invention may comprise more than one target sequence of one or more neuron-specific miRs, which may be identical or different. In a preferred embodiment of the invention, the viral vector comprises at least 2, 3, 4, 5, 6, 7, 8, 9 or 10 copies of a same target sequence of a neuron-specific miR according to the invention.

As intended here the RNA for silencing the gene may be of any type known by one of skill in the art. However, it is preferred that it is an antisens RNA, a small interfering RNA (siRNA), or a short hairpin RNA (shRNA), all of which are well known to one of skill in the art and target the mRNA of the gene to silence.

Preferably, the nucleic acid sequence encoding a RNA for silencing the gene according to the invention encodes a siRNA or a shRNA targeting the gene mRNA. More preferably, the shRNA is embedded in a miR sequence, preferably a miR30 sequence. miR-, in particular miR30-, embedding of a shRNA is well known to one of skill in art and notably comprises incorporating the shRNA within the flanking and loop sequences of microRNAs expressed in the cell type wherein expression of shRNA is intended to take place.

As intended herein, a viral vector relate to a particle derived from one or more viruses, in particular by suppression of one or more of its pathogenic or virulence determinants. Preferably, the viral vector according to the invention is selected from the group consisting of lentiviral vectors and adeno-associated vectors.

More preferably, the viral vector according to the invention is a lentiviral vector, most preferably a HIV-1 vector.

Preferably, the first and the second nucleic acid sequences according to the invention are on a same nucleic acid molecule. More preferably, the nucleic acid molecule according to the invention comprises, from 5' to 3':
- a 5' long terminal repeat (LTR) sequence, preferably represented by SEQ ID NO: 5,
- a sequence encoding a siRNA or shRNA targeting the gene, preferably embedded in a miR30 sequence,
- the GS rat promoter, preferably represented by SEQ ID NO: 3,
- a sequence encoding the tetracycline transactivator, preferably represented by SEQ ID NO: 1,
- the woodchuck hepatitis post-transcriptional regulatory element (WPRE), preferably represented by SEQ ID NO: 6,
- a sequence encoding 4 copies of a miR124 target sequence, preferably represented by SEQ ID NO: 7,
- a 3' long terminal repeat (LTR) sequence, in particular of self-inactivating (SIN) 3' LTR,
- the tetracycline response element (TRE).

Preferably, the 3'long terminal repeat (LTR) sequence, in particular the self-inactivating (SIN) 3' LTR, comprises the tetracycline response element (TRE), and is most preferably represented by SEQ ID NO: 8.

As intended herein, the 5' long terminal repeat (LTR) sequence may be a chimeric LTR sequence comprising a promoter, such as the CMV promoter, as is the case in SEQ ID NO: 5.

Preferably, the gene according to the invention is the huntingtin gene. In that case, the shRNA is preferably encoded by SEQ ID NO: 9 and the miR30-embedded shRNA is preferably encoded by SEQ ID NO: 10.

Thus, most preferably, where the vector is intended for silencing the huntingtin gene, the nucleic acid molecule according to the invention is represented by SEQ ID NO: 11 and the vector is represented by SEQ ID NO: 12.

Preferably, the viral vector according to the invention is for use in the prevention or treatment of astrocyte-mediated diseases.

The present invention also relates to a pharmaceutical composition comprising a viral vector according to the invention as an active ingredient, optionally in association with a pharmaceutically acceptable vehicle or excipient, in particular for use in the prevention or treatment of astrocyte-mediated diseases.

The present invention also relates to a method for preventing or treating astrocyte-mediated diseases in an individual, comprising administering a prophylactically or therapeutically effective amount of a viral vector according to the invention to the individual

As intended herein, "astrocyte-mediated diseases" in particular relate to diseases involving a dysfunction of astrocytes, more particularly a dysfunction due to the expression or the over-expression of a protein or glycoprotein. Astrocyte-mediated diseases according to the invention notably encompass Huntington's disease, Parkinson's disease and Alzheimer's disease.

The viral vector according to the invention is preferably formulated as a liquid formulation suitable for injection into the brain. Solutions for preparing a liquid formulation suitable for injection into the brain of a viral vector according to the invention are well known to one of skill in the art and notably comprise the TSSM formulation: 20 mM Tromethamine, 100 mM NaCl, 10 mg/ml sucrose and 10 mg/ml mannitol.

Viral vector administration may be performed by direct injection into the brain, for instance in the striatum. The viral vector is preferably administered at a dose of from 1x10⁵ transducing units (TU) to 5x10⁶ TU.

The present invention also relates to a method, in particular an *in vitro* method, for silencing a gene in astrocytes, comprising contacting a viral vector according to the invention with at least one astrocyte.

The present invention also relates to a nucleic acid molecule comprising from 5' to 3':
- a 5' long terminal repeat (LTR) sequence, preferably represented by SEQ ID NO: 5,
- a sequence comprising from 4 to 1000 nucleotides, such as a multiple cloning site or a sequence encoding a siRNA or a shRNA, preferably embedded in a miR30 sequence,
- the GS rat promoter, preferably represented by SEQ ID NO: 3,
- a sequence encoding the tetracycline transactivator, preferably represented by SEQ ID NO: 1,
- the woodchuck hepatitis post-transcriptional regulatory element (WPRE), preferably represented by SEQ ID NO: 6,
- a sequence encoding 4 copies of a miR124 target sequence, preferably represented by SEQ ID NO: 7,
- a 3'long terminal repeat (LTR) sequence, in particular a self-inactivating (SIN) 3' LTR,
- the tetracycline response element (TRE)

Preferably, the 3'long terminal repeat (LTR) sequence, in particular the self-inactivating (SIN) 3' LTR, comprises the tetracycline response element (TRE), and is most preferably represented by SEQ ID NO: 8.

As intended herein, the 5' long terminal repeat (LTR) sequence may be a chimeric LTR sequence comprising a promoter, such as the CMV promoter, as is the case in SEQ ID NO: 5.

Preferably, where it comprises a multiple cloning site, comprising the *Sph*I and the *Eco*RV restrictions sites, the nucleic acid molecule according to the invention is represented by SEQ ID NO: 13.

The present invention will now be further described by the following non-limiting Example and figures.

### Description of the figures

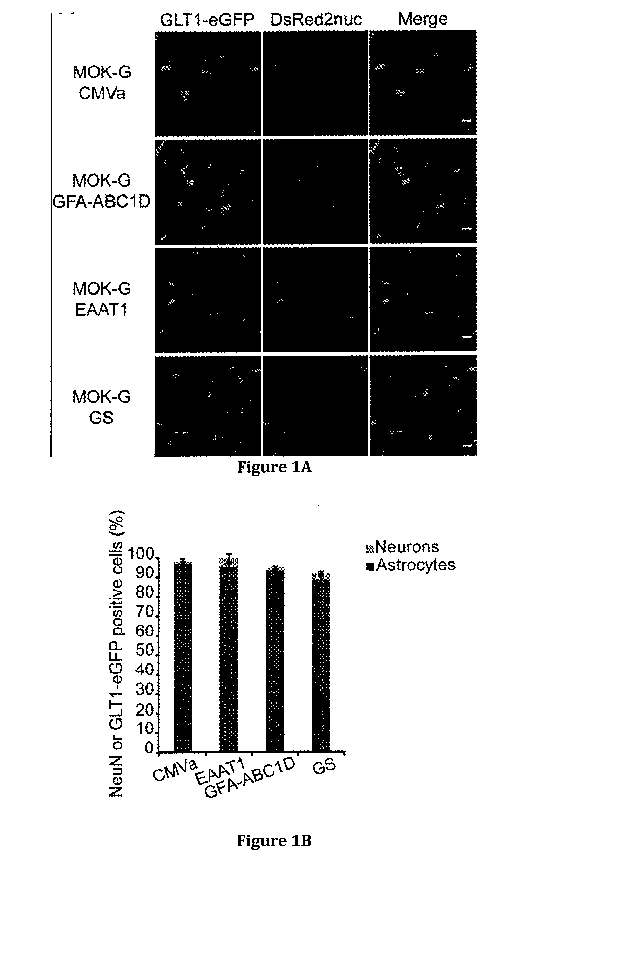

**Figures 1A and 1B****: Transduction efficiency and tropism in adult mice of lentiviral vectors containing the astrocytic promoters.** Figure 1A) MOK-G pseudotyped LVs carrying either CMVa, GFA-ABC1D, EAAT1 or GS were injected into striatum of BAC-GLT1-eGFP mice. Acquisition parameters were optimized for each promoter. Figure 1B) Quantification of DsRednuc-eGFP- and DsRednuc-NeuN-positive cells shows the astrocytic tropism of all vectors. Scale bar = 10 µm.

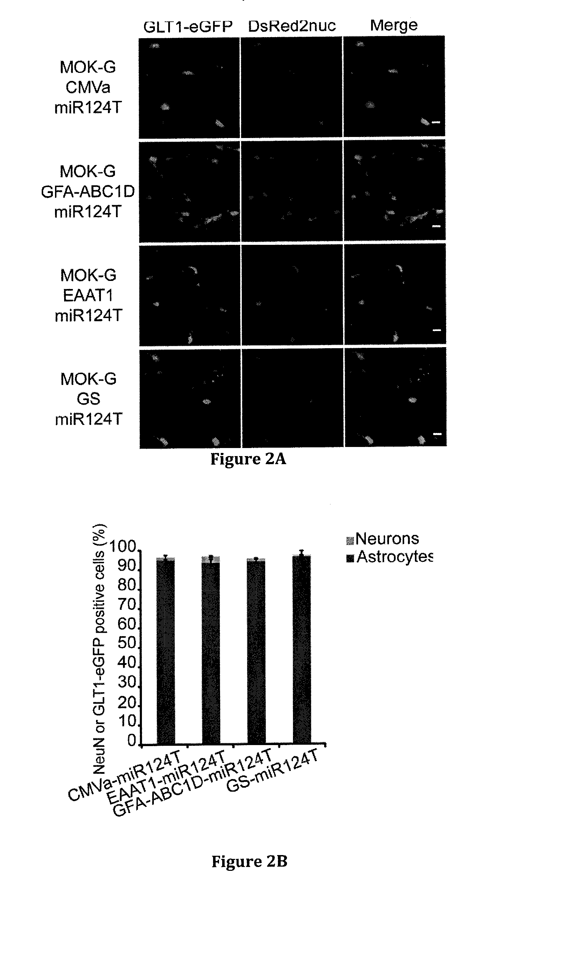

**Figures 2A and 2B****: Combinatorial effect of MOK-G and miR124T on lentiviral vectors tropism with astrocytic promoters.** Figure 2A) Double immunofluorescence staining for GLT1-eGFP and DsRednuc after the injection of MOK-G pseudotyped LVs into striatum of BAC-GLT1-eGFP. Acquisition parameters were optimized for each promoter. Figure 2B) Quantification of DsRednuc-eGFP- and DsRednuc-NeuN-positive cells shows the improvement of the astrocytic tropism for all vectors. Scale bar = 10 µm.

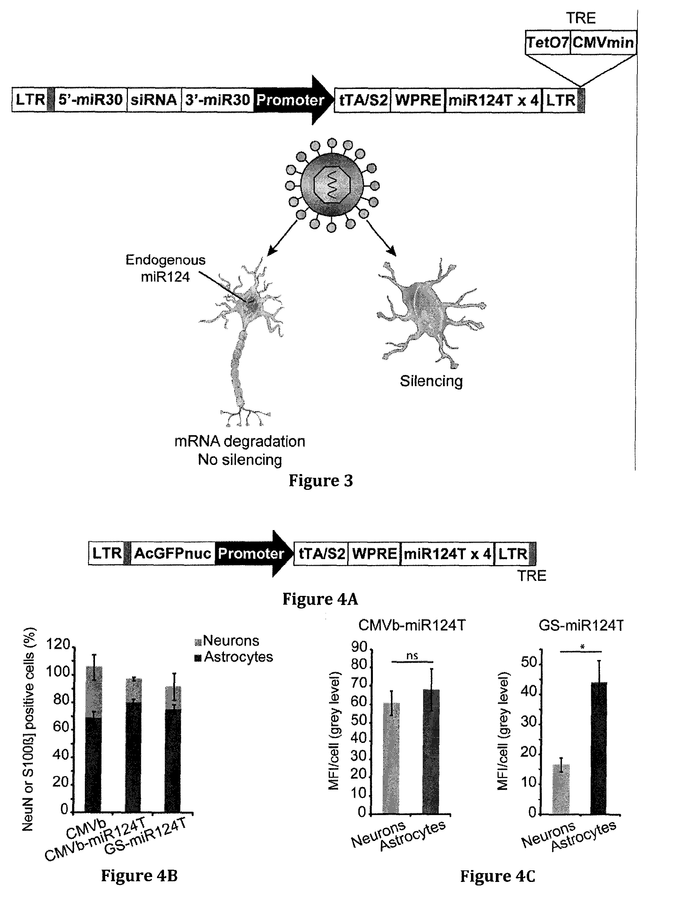

**Figure 3****: Principle of the astro-silencing lentiviral vector**. The schema represents the different elements composing the astro-silencing vector based on the use of the tetracycline-regulated system. If the LV enters into a neuron, the binding of miR124 to its target leads to the degradation of the mRNA, preventing for silencing. However, as astrocytes do not contain miR124, there is no mRNA degradation. Hence, theoretically, silencing should be observed only in astrocytes.
**Figures 4A, 4B and 4C****: Tropism of the astro-silencing lentiviral vector**. Figure 4A) To assess the tropism of the astro-silencing construct, a nuclear-localized GFP (AcGFPnuc) was used. MOK-G pseudotyped LVs were injected into striatum of BAC-GLAST-DsRed mice. As internal promoters, CMVb and GS were used. As control, a LV miR124T-less and containing CMVb was used. Figure 4B) Quantification of AcGFPnuc-DsRed-, AcGFPnuc-S100β-, AcGFPnuc-NeuN-positive cells shows a tropism preferentially astrocytic with CMVb-miR124T and GS-miR124T. Figure 4C) Measurement of the mean fluorescence intensity per cell (MFI/cell) highlights the astrocytic activity of GS compared to the ubiquitous CMVb.

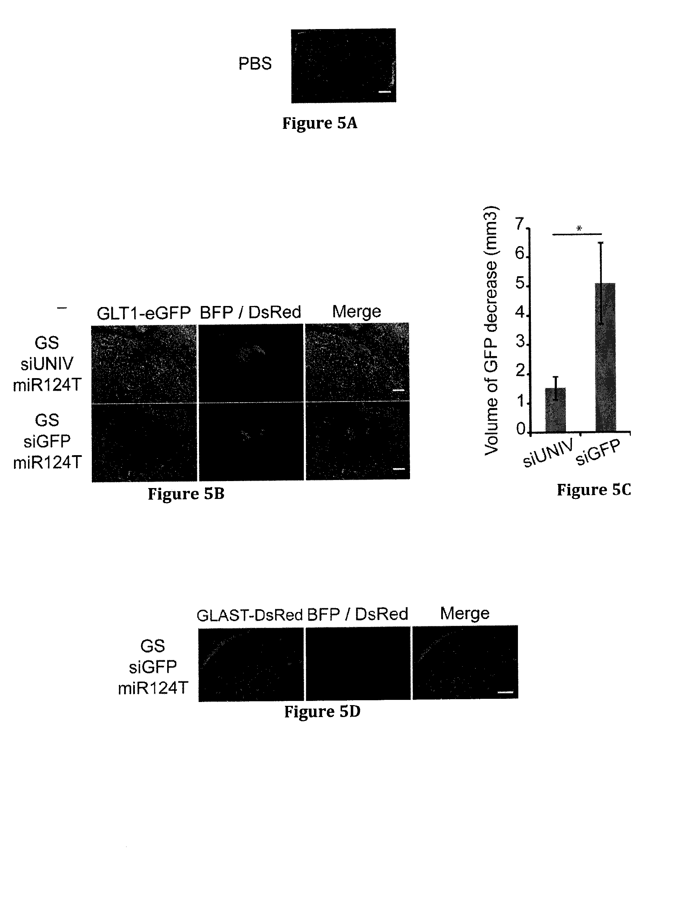

**Figures 5A, 5B, 5C and 5D****: Validation of the astro-silencing construct with a siGFP into BAC-GLT1-eGFP mice.** Figure 5A) Typical aspect of striatum BAC-GLT1-eGFP mice injected with PBS. Figure 5B) GS-siUNIV-miR124T and GS-siGFP-miR124T LVs have been injected into BAC-GLT1-eGFP striatum mice. To localize the injection site, BFP or DsRed have been co-injected with siUNIV and siGFP respectively. Injection of GS-siUNIV-miR124T induced a GFP decrease in the vicinity of BFP whereas injection of GS-siGFP-miR124T induced a large GFP decrease. Figure 5C) The measurement of the GFP decrease volume shows a significant difference between siGFP and siUNIV, confirming the efficacy of the siGFP. Figure 5D) GS-siGFP-miR124T LV has also been injected into BAC-GLAST-DsRed striatum mice to confirm that siGFP does not act on promoter to silence the transgene. Scale bar = 200 µm.

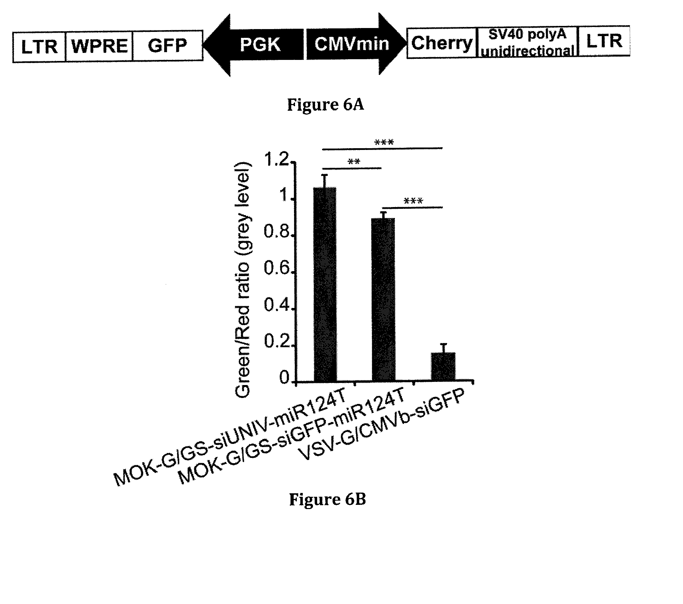

**Figures 6A and 6B****: Specificity of the astro-silencing construct.** Figure 6A) Bicistronic vector encoding Cherry and GFP under the control of CMV minimal and PGK promoters, respectively. This LV has been pseudotyped with VSV-G in order to transduce only neurons. Figure 6B) Quantification of Green/Red ratio reflects the silencing of GFP. LV-siUNIV (first column) is the internal control and represents the basal level of Green/Red ratio. As a control of the system efficiency, we have used a VSV-G pseudotyped LV encoding the siGFP under the control of the CMVb (third column) in order to target neurons. A significative Green/Red ratio is then observable. When the astro-silencing construct is used (second column), a slight decrease of Green/Red ratio is also observable, reflecting a low tropism for neurons.

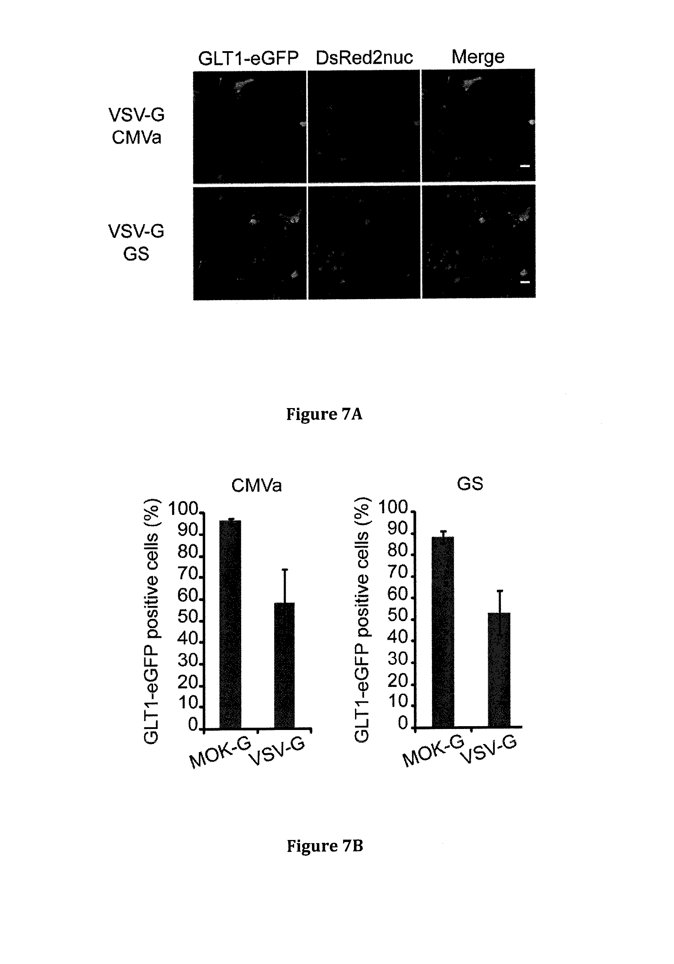

**Figures 7A and 7B****: Tropism of lentiviral vectors with a VSV-G envelope and astrocytic promoters.** Figure 7A) Double immunofluorescence staining for GLT1-eGFP and DsRednuc after the injection of VSV-G pseudotyped LVs into striatum of BAC-GLT1-eGFP. Acquisition parameters were optimized for each promoter. Figure 7B) Quantification of DsRednuc-eGFP-positive cells shows a partial astrocytic tropism with VSV-G pseudotyped LVs with CMVa or GS. Scale bar = 10 µm.

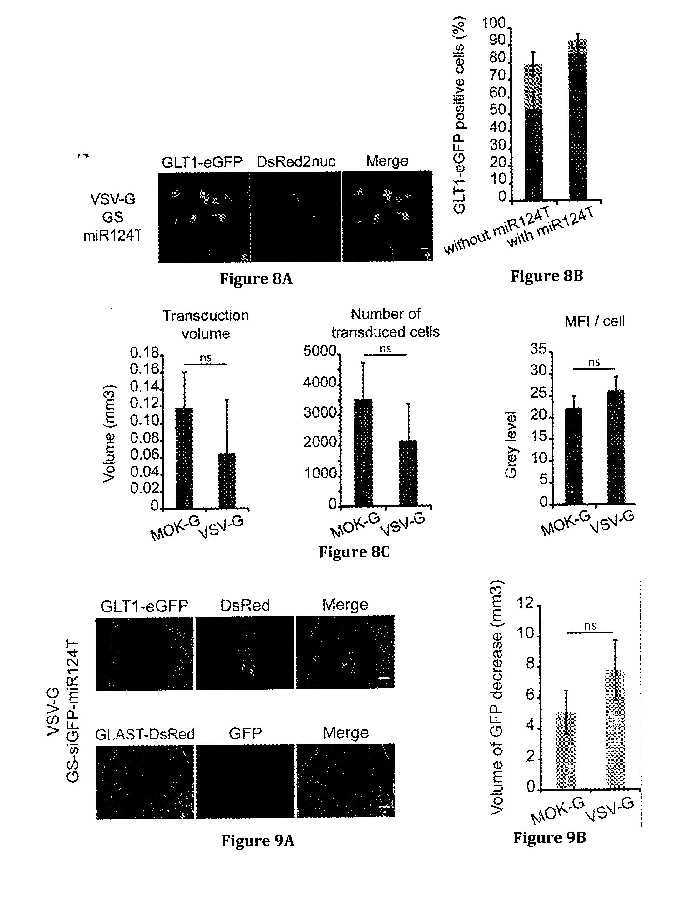

**Figures 8A, 8B and 8C****: Tropism of lentiviral vectors with a VSV-G envelope and GS promoter combining with miR124T.** Figure 8A) Double immunofluorescence staining for GLT1-eGFP and DsRednuc after the injection of VSV-G pseudotyped LV GS-DsRednuc-miR124T into striatum of BAC-GLT1-eGFP. Figure 8B) Quantification of DsRednuc-eGFP-and DsRednuc-NeuN-positive cells shows an astrocytic tropism with miR124T compared to LV without miR124T. Scale bar = 10 µm. Figure 8C) Comparison of LVs characteristics (i.e. transduction volume, number of transduced cells and MFI/cell) when pseudotyped by either MOK-G or VSV-G highlights no significant differences.
**Figures 9A and 9B****: Validation of the astro-silencing construct with a VSV-G envelope.** Figure 9A) GS-siGFP-miR124T LV pseudotyped with VSV-G has been injected into BAC-GLT1-eGFP and BAC-GLAST-DsRed striatum mice; to localize the injection site, DsRed or GFP have been co-injected respectively. Figure 9B) The measurement of the GFP decrease volume shows no significant difference between the two envelopes, MOK-G and VSV-G. Scale bar = 200 µm.

### EXAMPLE

In the present experimental setting, the tissue-specific promoter is driving the expression of the tetracycline transactivation and the miRT is further restricting the expression of the siRNA in astrocytes.

As tissue-specific promoters, the inventors have analysed three astrocytic promoters: the one of the excitatory amino acid transporter 1 (EAAT1), the glutamine synthetase (GS) and the shortest form of the GFAP promoter (GFA-ABC1D). These promoters were first tested in a constitutive system and then integrated in a single regulated vector previously described (Vigna *et al.,* 2005). Recent studies have demonstrated that siRNA embedded in a miRNA backbone may provide safer therapeutic for siRNA expression vectors, as compared to small hairpin RNAs, by decreasing the risk of saturation of the endogenous siRNA machinery (Boudreau *et al.,* 2009). In addition pol II promoters, such as tissue-specific promoters, are used to express miR-embedded siRNA (Boudreau *et al.,* 2009; Boudreau *et al.,* 2008; McBride *et al.,* 2008). As a proof-of-principle, the inventors have used a siRNA directed against the green fluorescent protein (GFP) reporter gene (siGFP). This siGFP has been embedded in miR30 (Boudreau *et al.,* 2009; McBride *et al.,* 2008); the design is such that the final mature siRNA is identical to the original H1-shRNA cassette (Boudreau *et al.,* 2008). Finally, the inventors have compared VSV-G and MOK-G envelopes to target astrocytes when combined with a tissue-specific promoter and with or without the miR124T sequence. To facilitate the analysis, BAC-GLT1-eGFP mice, which express GFP exclusively in astrocytes (GLT1 is specific of astrocytes) have been used (Regan *et al.,* 2007).

### MATERIALS AND METHODS

### Plasmids

For the tissue-specificity study, the inventors used self-inactivated (SIN) lentiviral vectors (LV) (Deglon *et al.,* 2000) containing the woodchuck post-regulatory element (WPRE, W), encoding the nuclear-localized red fluorescent protein (pDsRed2-Nuc, Clontech, Saint-Germain-en-Laye, France) and with or without 4 copies of the synthetic target of the miR124 (miR124T coding sequence: TAAGGCACGCGGTGAATGCCA (SEQ ID NO: 4); SIN-W-DsRed2nuc and SIN-W-DsRed2nuc-miR124T).

The following internal promoters were used: the ubiquitous mouse phosphoglycerate kinase 1 promoter (PGK; from -430 to +74 relative to the transcriptional start site (TSS); GenBank M18735.1 nt: 423 to 931) (Adra *et al.,* 1987; Deglon *et al.,* 2000) and the human cytomegalovirus (688 bp CMVa from pcDNA3.1 plasmid, Clontech, Saint-Germain-en-Laye, France; GenBank K03104, nt: 137 to 723 and sequences with a putative transcriptional start site, T7 primer binding site and multiple cloning sites). A slightly different fragment of the human cytomegalovirus promoter was cloned in the inducible vector (606 bp CMVb; GenBank K03104, nt: 207 to 791).

The following promoters the inventors used for tissue-specific expression: the 2 kb human excitatory amino acid transporter 1 (EAAT1; synthesized by GeneArt (Invitrogen, Cergy-Pontoise, France; GenBank AF448436.1, nt: 1 to 2051) promoter (Kim *et al.,* 2003); the shortest form, 681 pb, of the human glial fibrillary acidic protein (pGFA-ABC1D; courtesy of Dr Brenner, Birmingham, USA; GenBank NG_008401.1, nt: 3292 to 3793 and 4916 to 5094) promoter (Lee *et al.,* 2008); and the 432 pb rat glutamine synthetase (GS; GenBank M91651.1, nt: 2111 to 2543) promoter which was amplified from rat genomic DNA with the following primers: 5'-CACCATCGATGGCTCGCTCAACAAAGGGTAA-3' (SEQ ID NO: 14) and 5'-GGATCCCTCGGCTGTGGAGGGTTGCGG-3' (SEQ ID NO: 15) (GenBank M91651.1, nt: 1 to 2132 with a CACC flanking sequence for oriented cloning - and nt: 2524 to 2543 for forward and reverse primer, respectively) (Mill *et al.,* 1991).

For the silencing study, the inventors used a one-single inducible system (courtesy of Prof Naldini, Milano, Italy) (Vigna *et al.,* 2005), self-inactivated LV with an internal cytomegalovirus promoter (pCCL) and containing: i) the transactivator sequence (tTA/S2), ii) the tetracycline response element (TRE), iii) the central polypurine tract (cPPT), iv) the GS promoter or the human cytomegalovirus promoter (CMVb; GenBank AY468486.1, nt: 2020 to 2624), v) the WPRE, vi) four copies of miR124T, and vii) a multiple cloning site (MCS) (pCCL-MCS-cPPT-GS-tTA/S2-W-miR124T-TRE-SIN). To assess the tissue-specificity of such a vector, they first cloned the nuclear-localized green fluorescent protein (pAcGFPI-Nuc; Clontech, Saint-Germain-en-Laye, France) by a NheI restriction enzyme digestion into the MCS (pCCL-AcGFPnuc-cPPT-GS-tTA/S2-W-miR124T-TRE-SIN). To evaluate the silencing potential of the vector, the inventors used an siRNA directed against the green fluorescent protein (siGFP; Drouet *et al.* 2009) and as control a siRNA with no sequence homology in the genome (siUNIV). These siRNAs were embedded in a miR30 5' and 3' flanking regions and synthesized by GeneArt (Invitrogen, Cergy-Pontoise, France). A SacI-KpnI fragment was excised from the pENTR plasmid, end-filled with T4 DNA polymerase (Invitrogen, Cergy-Pontoise, France) and inserted as a blunt-ended fragment into the destination plasmid (pCCL-miR30-siRNA-cPPT-GS-tTA/S2-W-miR124T-TRE-SIN). To localize the injection site, the inventors used LVs expressing reporter genes under the control of the mouse PGK: either the DsRed (SIN-W-PGK-DsRed) or the blue fluorescent protein (BFP, SIN-W-PGK-BFP).

To evaluate the specificity of our silencing contructs, the inventors used a bicistronic vector (pCCL-mCherry-CMVmin-hPGK-eGFP-WPRE-SIN; courtesy of Prof Naldini, Milano, Italy) containing: i) the red fluorescent protein (Cherry) reporter gene under the control of a minimal CMV promoter, ii) the GFP reporter gene under the control of the mouse PGK promoter, iii) the WPRE, and iv) an unidirectional polyA signal derived from the SV40. This LV encodes both Cherry and GFP.

### Lentiviral vector production

Lentiviral vectors were produced in 293T cells, using a four-plasmid system, as previously described (Hottinger *et al.,* 2000). The HIV-1 vectors were pseudotyped with either the vesicular stomatitis virus G protein (VSV-G) or a codon-optimized version of the G protein of Mokola lyssavirus (MOK-G) (pMOK-G; GeneArt). Viruses were concentrated by ultracentrifugation and resuspended in phosphate-buffered saline (PBS) / 1% bovine serum albumin (BSA). The viral particle content of each batch was determined by p24 antigen enzyme-linked immunosorbent assay (RETROtek; Gentaur, Paris, France). The stocks were stored at -80°C until use.

### Animals

Male C57BL/6 mice (weight, 20g; Iffa Credo/Charles River, France) and adult transgenic mice (BAC-GLT1-eGFP and BAC-GLAST-DsRed) (weight, around 20-25g; lineage perpetrated in our own animal house) (Regan *et al.,* 2007) were used. The animals were housed in a temperature-controlled room and maintained on a 12 h day/night cycle. Food and water were available *ad libitum.* All experimental procedures were performed in strict accordance with the recommendations of the European Community directive (86/609/EEC) concerning the care and use of laboratory animals.

### Stereotaxic injections

### Tissue-specificity study

Concentrated viral stocks were thawed on ice and resuspended by vortexing and repeated pipetting. SIN-W-promoter-DsRed2nuc LVs with or without the miR124T were diluted in PBS/1%BSA to a final concentration of 67 ng p24/µl. BAC-GLT1-eGFP mice were anesthetized by intraperitoneal injection with a mixture of 150 mg/kg ketamine and 10 mg/kg xylazine (Coveto, Montaigu, France). Suspensions of lentiviral vectors were injected into the brain using a 34-gauge blunt-tip needle linked to a Hamilton syringe by a polyethylene catheter. Stereotaxic coordinates for injection into mouse striatum were, from bregma: anteroposterior +1 mm; lateral +/-2 mm and ventral -2.5 mm from the dura, with tooth bar set at 0 mm (Franklin and Paxinos, 1997). Mice received a total volume of 3 µl of the vector preparation, administered at a rate of 0.2 µl/min. At the end of injections, needles were left in place for 5 min before being slowly removed. The skin was sutured and mice were allowed to recover.

### Silencing study

To assess the tropism of the silencing vector, pCCL-AcGFPnuc-cPPT-GS-tTA/S2-W-miR124T-TRE-SIN was diluted in PBS/1%BSA to a final concentration of 67 ng p24/µl. BAC-GLAST-DsRed mice were anesthetized by intraperitoneal injection with a mixture of 150 mg/kg ketamine and 10 mg/kg xylazine (Coveto, Montaigu, France). Suspensions of lentiviral vectors were injected into the brain using a 34-gauge blunt-tip needle linked to a Hamilton syringe by a polyethylene catheter. Stereotaxic coordinates for injection into mouse striatum were, from bregma: anteroposterior +1 mm; lateral +/-2 mm and ventral -2.5 mm from the dura, with tooth bar set at 0 mm (Franklin and Paxinos, 1997). Mice received a total volume of 3 µl of the vector preparation, administered at a rate of 0.2 µl/min. At the end of injections, needles were left in place for 5 min before being slowly removed. The skin was sutured and mice were allowed to recover.

For evaluating the specificity of the silencing vector, the bicistronic LV was diluted in PBS/1%BSA to a final concentration of 33 ng p24/µl. C57BL/6 mice were anesthetized and injected as described above. Mice received a total volume of 3 µl of the vector preparation, administered at a rate of 0.2 µl/min. At the end of injections, needles were left in place for 5 min before being slowly removed. The skin was sutured and mice were allowed to recover.

For the silencing, pCCL-miR30-siGFP or miR30-siUNIV-cPPT-GS-tTA/S2-W-miR124T-TRE-SIN were diluted in PBS/1%BSA to a final concentration of 67 ng p24/µl; localization LVs SIN-W-PGK-DsRed and SIN-W-PGK-BFP were diluted to a final concentration of 17 ng p24/µl. BAC-GLT1-eGFP mice were anesthetized and injected as described above. Mice received a total volume of 3 µl of the vector preparation, administered at a rate of 0.2 µl/min. At the end of injections, needles were left in place for 5 min before being slowly removed. The skin was sutured and mice were allowed to recover.

### Histological processing

### Tissue preparation

Two (silencing study) or three weeks (tissue-specificity study) post-lentiviral injection, the animals were killed with an overdose of sodium pentobarbital and transcardially perfused with a 4% paraformaldehyde (PFA; Sigma-Aldrich, Saint-Quentin Fallavier, France) solution. The brains were removed and post-fixed by incubation in 4% PFA for about 12 h and then cryoprotected first in 15% sucrose / 0.1 M PBS overnight and then in 30% sucrose / 0.1 M PBS for 24 h. A sledge microtome with a freezing stage at -30 °C (SM2400; Leica, Nanterre, France) was used to cut brain coronal sections 30 µm thick. Slices throughout the entire striatum were collected and stored in tubes as free-floating sections in PBS supplemented with 0.12 µM sodium azide. Tubes were stored at 4 °C until immunohistochemical processing.

### Primary antibodies

The following primary antibodies were used: mouse monoclonal anti-neuronal nuclei antibody (NeuN, dilution 1/200; MAB377, Millipore, Molsheim, France); mouse monoclonal antibody recognizing the β subunit of the S100 protein (S100β, dilution 1/500; S2532, Sigma-Aldrich, Saint-Quentin Fallavier, France).

### Immunohistochemical procedure

For the tissue-specificity study and the silencing study, one serial was mounted directly in an aqueous medium (FluorSave, Life Technologies, Saint Aubin, France).

For the study of SIN-W-promoter-DsRed ± miR124T tropism, sections from BAC-GLT1-eGFP mice were labeled with NeuN antibody. Staining was performed with the mouse-on-mouse detection kit Vector ® M.O.M. kit (Basic) (Clinisciences, Nanterre, France). As secondary antibody, the AlexaFluor 350 (blue) anti-mouse diluted 1/500 (Life Technologies, Saint Aubin, France) was used.

For the tropism of pCCL-AcGFPnuc-cPPT-GS-tTA/S2-W-miR124T-TRE-SIN, two serials from BAC-GLAST-DsRed mice were labeled with either NeuN or S100β antibodies also by using the basic M.O.M. kit (Clinisciences, Nanterre, France). As secondary antibody, the AlexaFluor 350 (blue) anti-mouse diluted 1/500 (Life Technologies, Saint Aubin, France) was used.

### Quantitative analysis

### Co-localization with astrocytic or neuronal markers

Sections labeled for GLT1-eGFP, NeuN or S100β were analyzed by epifluorescence microscopy with a Leica DM6000B (Leica, Nanterre, France) microscope equipped with an automated motorized stage and image acquisition software (MorphoStrider, Explora nova, La Rochelle, France).

For the tissue-specificity study, the numbers of DsRed2nuc-NeuN-positive cells and DsRed2nuc-GLT1-eGFP-positive cells were determined on images acquired with a 40x objective (six animals, three sections per animal, six images per section) by ImageJ software (http://rsb.info.nih.gov/ij/, NIH, USA).

To assess the tropism of pCCL-AcGFPnuc-cPPT-GS-tTA/S2-W-miR124T-TRE-SIN, the numbers of AcGFPnuc-NeuN-positive cells and AcGFPnuc-S100β-positive or AcGFPnuc-GLAST-DsRed-positive cells were determined on images acquired with a 40x objective (six animals, three sections per animal, six images per section) by ImageJ software (http://rsb.info.nih.gov/ij/, NIH, USA).

### Mean fluorescence intensity per cell

For the silencing study, to quantify the mean fluorescence intensity per cell (MFI/cell) in neurons and in astrocytes, photomicrographs of AcGFPnuc-positive BAC-GLAST-DsRed mice sections, stained with NeuN or S100β, were acquired with a 40x objective (Morphostrider software; Explora Nova, La Rochelle, France). The acquisition parameters of the Leica DM6000B microscope (excitation neutral attenuator filters and obturator) were maintained equivalent for all acquisitions while camera parameters (time of exposure from 300 to 900 ms, and gain from 1 to 3) were adjusted for each group (e.g. pCCL-AcGFPnuc-cPPT-GS-tTA/S2-W-miR124T-TRE-SIN and pCCL-AcGFPnuc-cPPT-CMVb-tTA/S2-W-miR124T-TRE-SIN) to maximize fluorescence but without saturation (avoided using display of pixel fluorescence intensity histogram). Co-localization with NeuN or S100β was used to delimite the neurons (70 on average for each group) and the astrocytes (400 in average for each group) and the ImageJ software automatically calculated the mean gray level of the various objects drawn. To allow semi-quantitative comparison between the constructs, fluorescence levels for each group (construct) was normalized to the same time exposure (500 ms, gain 1). The inventors experimentally checked that fluorescence emission (gray levels) GFP containing brain sections was linearly proportional to exposure time in the range they used (300-900 ms) and for the different gains used.

For the comparison of VSV-G and MOK-G envelopes with SIN-W-GS-DsRed2nuc-miR124T, photomicrographs of DsRednuc-positive BAC-GLT1-eGFP mice sections were acquired with a 10x objective. Mosaic pictures covering the entire striatum were generated (Morphostrider software; Explora Nova, La Rochelle, France) and used to measure the fluorescence intensity. The acquisition parameters of the Leica DM6000B microscope (excitation neutral attenuator filters and obturator) were maintained equivalent for all acquisitions as the camera parameters. The section closest to the injection site was used to measure the MFI/cell. The DsRednuc-positive area was delimited and a gray level threshold was applied for automated segmentation and count of infected cells expressing GFP (the lower limit of the gray level was set-up at 9). The ImageJ software automatically calculated the number of transduced cells and the mean gray level of the various objects identified. To determine the transduction volume, DsRednuc-positive area was delimited for each section and the volume calculated based on this data as we know that at 10x objective, 1 pixel represents 0.643299 µm.

To measure the silencing level in neurons with the bicistronic vector, photomicrographs of the GFP-Cherry-positive C57BL/6 mice sections were acquired with a 10x objective. Mosaic pictures covering the entire striatum were generated (Morphostrider software; Explora Nova, La Rochelle, France) and used to measure the fluorescence intensity for each reporter gene. The acquisition parameters of the Leica DM6000B microscope (excitation neutral attenuator filters and obturator) were maintained equivalent for all acquisitions as the camera parameters. GFP-positive and Cherry-positive areas were delimited and the mean fluorescence intensity automatically calculated (Mercator software; Explora Nova, La Rochelle, France). Green/Red ratio was then calculated.

### Statistical analysis

Data were analyzed using GraphPad Prism 4 Software (GraphPad Software, La Jolla, USA). A paired *t* test analysis was used to evaluate the significance differences of MFI/neuron *vs* MFI/astrocyte (set at *P* < 0.05). Unpaired *t* test analysis were used to appraise the significance differences of the volume of GFP decrease (siUNIV *vs* siGFP and VSV-G *vs* MOK-G) in the silencing study and to compare the features of VSV-G and MOK-G pseudotyped LVs (set at *P* < 0.05). One-way ANOVA with a post-hoc Newman-Keuls test analysis was used to assess the significance of differences (set at *P* < 0.05) for the study of specificity based on the use of the bicistronic vector. Data are presented as means ± standard error of the mean (SEM).

### RESULTS

### Effect of astrocytic promoters on LVs tropism

As a first step toward the establishment of an astrocyte-specific silencing, the inventors evaluate three astrocytic promoters. The human glial fibrillary acidic protein (GFAP), the human excitatory amino acid transporter 1 (EAAT1) and the rat glutamine synthetase (GS) were cloned upstream of a nuclear DsRed (SIN-W-DsRed2nuc) and pseudotyped with the MOK-G envelope. As controls, LVs with the housekeeping PGK and CMVa promoters (SIN-W-PGK/CMVa-DsRed2nuc) and a VSV-G envelope were used (Deglon *et al.,* 2000). The constructs were injected into striatum of adult BAC-GLT1-eGFP mice, and three weeks later the mice were sacrificed and sections of the striatum were processed for immunofluorescence. DsRed was detected in nuclei around the injection site in all animals; no fluorescence was detected in the untreated contralateral striatum. Antibody against the neuronal marker NeuN and the endogenous GLT1-eGFP, expressed only in astrocytes, were used to determine the phenotype of the transduced cells (Fig. 1). Microscopic analysis and cell counts revealed that for all astrocytic promoters, more than 90% of the transduced cells co-localized with GLT1-eGFP, whereas only scarce DsRed2nuc-positive cells were double-stained for NeuN (Fig. 1). Thus, all these promoters retained an astrocytic specificity. Interestingly, CMVa leads to the same results as observed with astrocytic promoters, confirming its transcriptional pattern to be more astrocytic than PGK: 92% of DsRed-positive cells were astrocytes on average, 96.4 ± 1.1% for CMVa and only 62.9 ± 2.7% for PGK. LV PGK specifically targets neurons with more than 90% of co-localization with the marker NeuN; whereas, pseudotyping with a MOK-G envelope shifts the tropism of the LV-PGK-DsRed2nuc with more than 70% of transduced cells co-localizing with GLT1-eGFP-positive astrocytes.

### Effect of the combination of an astrocytic promoter with the miR124T detargeting strategy on LVs tropism

To further improve the selectivity of the transgene expression, the inventors then used a detargeting strategy based on the addition of four copies of a synthetic miR124T sequence after the transgene. CMVa, EAAT1, GFA-ABC1D and GS promoters were inserted into SIN-W-DsRed2nuc-miR124T LVs and pseudotyped with MOK-G. The constructs were administered by injection into striatum of adult BAC-GLT1-eGFP mice. Three weeks later, mice were sacrificed and sections of the striatum were double-stained with the marker NeuN for co-localization analysis. Quantification of this co-localization between DsRed2nuc and either GLT1-eGFP or NeuN revealed a strong astrocytic tropism for all LVs tested: around 95% of DsRed2nuc positive cells were GLT1-eGFP-positive (Fig. 2). In particular, for GS promoter, there is a great improvement in targeting astrocytes by addition of the miR124T compared to LVs without miR124T (96.3 ± 2.5% and 88.2 ± 2.9%, respectively) (Fig. 1 and Fig. 2). Based on those results, we have chosen to pursue the studies with the GS promoter.

### Construction and tropism of the astro-silencing lentiviral vector

The next step in the development of an astrocyte-specific silencing was the integration of all these elements in a single tetracycline-regulated lentiviral backbone (Vigna *et al.,* 2005) (Fig. 3). Briefly, the strategy is based on the integration of four copies of the miR124T sequence on the 3'-end of the transactivator (tTA/S2) mRNA in order to prevent neuronal expression of the transgene. Moreover, to ensure an astrocytic expression, the tTA/S2-miR124T cassette is under the control of an astrocytic promoter. Hence, if LV enters into neurons, mRNA corresponding to tTA/S2-miR124T is degraded whereas if LV enters into astrocytes, there is no degradation and tTA/S2 binds to the tetracycline response element (TRE) and activate the CMV minimal promoter.

Based on previous results, the inventors have placed tTA/S2 under the control of either the GS promoter or CMVb as control (Fig. 4) and added four copies of the miR124T. The tropism of LVs was assessed with a nuclear GFP (AcGFPnuc) reporter gene. The vectors were pseudotyped with MOK-G and injected into striatum of adult BAC-GLAST-DsRed mice. Quantitative analysis of NeuN- and S100β- positive cells three weeks post-injection in the striatum confirmed the importance of the presence of miR124T with 69 ± 4.6% for CMVb against 80.3 ± 2.3% for CMVb-miR124T of double-stained astrocytes (Fig. 4B). With GS promoter combined with miR124T, 75.2 ± 3.5% of astrocytes are transduced (Fig. 4B).

To further quantify the residual transcriptional activity in neurons, the mean fluorescence intensity per cell (MFI/cell) was measured. The inventors show that with the GS promoter, a lower MFI/cell was observed in neurons compared with astrocytes (16.6 ± 2.3 *vs* 44.2 ± 7.1 grey level, respectively; *P* = 0.0347, paired *t* test) (Fig. 4C) whereas with the CMVb, there was no significant difference in transcriptional activity between neurons and astrocytes (60.6±6.7 *vs* 68.0±11.5 grey level, respectively; *P* = 0.0885, paired *t* test) (Fig. 4C). Hence, it confirms the interest of the GS promoter in this configuration.

### Validation of the astro-silencing construct with a siGFP

To validate the efficacy of the astro-silencing, the inventors cloned a siRNA directed against the GFP (siGFP). As control, they have used a universal siRNA (siUNIV), a negative control designed to have no homology to known gene sequences. The LVs-W-GS-siGFP/siUNIV were pseudotyped with MOK-G. They have injected them in adult BAC-GLT1-eGFP mice. To visualize the injection site, they have co-injected a LV expressing the BFP or the DsRed, respectively. Two weeks later, mice were sacrificed and direct fluorescence of striatum sections observed (Fig. 5). The inventors can clearly see a decrease of the GFP expression in animals injected with the siGFP in the red area (Fig. 5B-C); whereas, for animals injected with the siUNIV, such a silencing is not observed (Fig. 5B-C). Measure of GFP-less area confirms those observations. The volume of GFP decreases significantly more with the siGFP than with the siUNIV (5.1 ± 1.4 mm³ *vs* 1.5 ± 0.4 mm³; unpaired *t* test, *P* = 0.0161) (Fig. 5C).

To exclude a potential non-specific loss of GFP expression due to either the surgical procedure or the injection of viral particles, the inventors have tested this construct in BAC-GLAST-DsRed mice. They have not observed any alteration of DsRed expression (Fig. 5D) confirming the efficacy of the silencing.

### Specificity of the astro-silencing construct

To test the selectivity of their astro-silencing construct (i.e. absence or residual silencing in neurons), the inventors have used a vector carrying a bicistronic cassette and expressing both the Cherry and the GFP reporter genes in all infected cells (LV-Cherry-GFP) (Fig. 6A). The LV-Cherry-GFP was pseudotyped with VSV-G to transduce neurons (Naldini *et al.,* 1996). In this context, two scenario could occur: i) a decrease in Cherry/GFP fluorescent ratio reflecting a residual silencing of GFP expression in neurons or ii) no change in Cherry/GFP fluorescent ratio demonstrating the tissue-specificity of the silencing.

Adult C57/b16 mice were co-injected with the LV-Cherry-GFP and either the LV-GS-siUNIV or the LV-GS-siGFP. Two weeks later, mice were sacrificed and striatum sections analyzed. The green/red ratio obtained with the control siUNIV (1.06 ± 0.07) represents the reference for the experiment. When the inventors used, as positive control, LV-CMVb-siGFP pseudotyped with VSV-G (neuronal condition), the green/red ratio is drastically decreased (0.15 ± 0.05; *P*<0.001, one-way ANOVA with Newman-Keuls *post-hoc* test). When LV-Cherry-GFP is co-injected with LV-GS-siGFP, the green/red ratio (0.89±0.03) is slightly decreased by 16% (P < 0.01, one-way ANOVA with Newman-Keuls *post-hoc* test) compared to the control LV-GS-siUNIV (Fig. 6). These results are in agreement with residual activity of our astro-silencing construct in neurons (Fig. 4).

### Impact of pseudotyping and promoters on VSV-G tropism

The inventors have re-investigated the paradigm of the promoter-dependence tropism of VSV-G pseudotyped LVs. Indeed, some studies have shown that LVs pseudotyped with VSV-G and carrying an astrocytic promoter (such as GFAP or CMV) lead to an astrocytic tropism (Jakobsson *et al.,* 2003; Meunier *et al.,* 2008; Miletic *et al.,* 2004).

Therefore, they have used PGK, CMVa and GS which were inserted into SIN-W-DsRed2nuc. VSV-G pseudotyped LVs were administered into adult BAC-GLT1-eGFP mice, and three weeks later, mice were sacrificed and striatum sections processed for fluorescence immunostaining. They first assess the tropism by quantify the co-localization between DsRed2nuc and either NeuN or GLT1-eGFP staining (Fig. 7). LV carrying PGK was shown to be highly neurotropic. Interestingly, LVs with either CMVa or GS show a partial astrocytic tropism (58.3±15.2% and 52.8 ± 10.3%, respectively) (Fig. 7). These data confirm the promoter-dependence of VSV-G envelope when compared to results obtained with the MOK-G envelope.

### miR124T detargeting strategy and VSV-G tropism

To further improve the astrocytic tropism of VSV-G, the inventors added the miR124T to SIN-W-GS-DsRed2nuc. BAC-GLT1-eGFP mice were injected and sacrificed three weeks later. Co-localization analysis shows that addition of miR124T enhances astrocytic tropism by 1.6-fold (52.8 ± 10.3% without miR124T *vs* 85 ± 8.2% with miR124T) (Fig. 8). Furthermore, they have compared VSV-G and MOK-G pseudotyped LVs for three main features important for *in vivo* experiments: i) the transduction volume, ii) the number of transduced cells, and iii) the MFI/cell. No significant differences were measured, although a weak tendency seems to appear in favor of MOK-G concerning the two first points (Fig. 8). Finally, they have evaluated the potential of VSV-G pseudotyped LVs to silence specifically a transgene in astrocytes. LV-GS-siGFP was pseudotyped with VSV-G and co-injected into BAC-GLT1-eGFP mice with a LV expressing the DsRed to localize the injection area. Two weeks later, mice were sacrificed and striatum sections directly observed for endogenous fluorescence. As observed with LV-GS-siGFP pseudotyped with MOK-G, they have obtained a large GFP-less volume (Fig. 9). This consolidates the previous results and substantiates the potency of VSV-G pseudotyped LVs for astro-specific silencing.

### DISCUSSION

Tools allowing local and cell-type specific silencing is an essential prerequisite to further reveal cell-autonomous functions and identify processes that depend on cell-cell interactions in neurodegenerative diseases. In the present study the inventors developed a system to selectively silence a ubiquitous gene in astrocytes, based on MOK-G- or VSV-G-LVs carrying an astrocytic promoter, the miR124T sequence and an inducible system.

Previous studies have shown that MOK-G pseudotyped LVs with a CMV promoter have a partial tropism for astrocytes (Cannon *et al.,* 2010; Desmaris *et al.,* 2001; Pertusa *et al.,* 2008; Watson *et al.,* 2002). Although CMV is a ubiquitous promoter, it has been shown to be more astrocytic than neuronal (Jakobsson *et al.,* 2003; Li *et al.,* 2010; Meunier *et al.,* 2008) may be as a consequence of the inhibition of CMV neuronal activity by surrounding astrocytes (Kugler *et al.,* 2001; Sarkis *et al.,* 2000). Hence, a partial astrocytic targeting could be achieved with MOK-G pseudotyped LVs and an ubiquitous promoter.

To improve the tissue-specific expression, the inventors compare MOK-G pseudotyped LVs carrying three astrocytic promoters. The tropism was investigated in transgenic mice expressing the GFP reporter gene under the control of the glutamate transporter 1 (GLT1 *aka* solute carrier family 1 member 2, Slcla2) promoter (Regan *et al.,* 2007). GLT1 is the dominant glutamate transporter throughout the forebrain and the spinal cord (Berger and Hediger, 1998; Furuta *et al.,* 1997) and has been considered as an astroglial protein (Chaudhry *et al.,* 1995; Lehre *et al.,* 1995; Rothstein *et al.,* 1994; Torp *et al.,* 1994; Yang *et al.,* 2011). Nonetheless, GLT1 promoter has also low neuronal activity in specific area of the brain (Chen *et al.,* 2002; de Vivo *et al.,* 2010; Mennerick *et al.,* 1998); notably in the neocortex and the hippocampus (de Vivo *et al.,* 2010). However, the neuronal expression of GLT1 is observed during development or after injury (Martin *et al.,* 1997; Mennerick *et al.,* 1998; Northington *et al.,* 1999). When injected in the striatum of adult mice, more than 92% of tranduced cells expressing the DsRednuc reporter gene co-localized with GLT1-GFP. These data demonstrate that EAAT1, GFA-ABC1D and GS promoters improve the tropism of LV, as previously reported for the GFAP promoter (Jakobsson *et al.,* 2003; Kuroda *et al.,* 2008; Liu *et al.,* 2008).

The addition of the miR124T sequence further improves this astrocytic targeting (Colin *et al.,* 2009). The miR124T target sequence acts at the post-transcriptional level, by inducing the degradation of the mRNA (Brown *et al.,* 2007b; Brown *et al.,* 2006). This detargeting effect is particularly evident for GS. Indeed, albeit GS is highly expressed in astrocytes and in liver, the promoter is also active at a lower level in neurons (Mearow *et al.,* 1989; Mill *et al.,* 1991). Thus, addition of miR124T sequence in the LV backbone prevents the neuronal expression of the transgene. The inventors then combined this GS-miR124T with the tetracycline regulated system (Vigna *et al.,* 2005). First of all, they assessed the tropism of this construct with the nuclear-localized GFP and shown that if they target preferentially astrocytes, the residual expression in neurons was higher than with the constitutive system. Similar results were obtained with a siRNA targeting the GFP. One hypothesis to explain this difference is that the tetracycline system is creating amplification loop, which enhances transgene expression (Blesch *et al.,* 2005; Regulier *et al.,* 2002). So, it is conceivable in the constitutive system the weak GS transcriptional activity was not associated with detectable transgene expression in neurons whereas in the inducible system, residual reporter gene expression was measured. One possibility to overcome this partial leakiness of the system would be to incorporate not only miR124T sequence but combine several miRT to provide an additive or synergistic effects as previously reported (Brown *et al.,* 2007b; Doench *et al.,* 2003). Indeed, combinatorial arrangement of tissue-specific miRNA-targets efficiently suppresses transgene expression (Brown *et al.,* 2007b). Therefore, the use of several neuronal-specific miRNAs, such as the miR124 and miR10 (Smith *et al.,* 2010) could be considered to prevent more effectively the neuronal transgene expression. As an alternative, one could consider to use an astrocytic minimal promoter instead of the human CMV minimal promoter in the tetracycline system. The use of a glial core promoter, such as the core promoter of the human glutamate carboxypeptidase II or the human JC papovavirus promoter (Han *et al.,* 2007; Krebs *et al.,* 1995), instead of a CMV core promoter should prevent neuronal expression. Finally, a better characterization of astrocytes-specific promoters may further improve tissue-specific expression in the brain (ENCODE and Pleiade projects) (D'Souza *et al.,* 2008; ENCODE, 2004; Portales-Casamar *et al.,* 2010; Yang *et al.,* 2009).

To test the silencing system, the inventors have used a siRNA directed against the GFP reporter gene embedded in a miR30 sequence (Shin *et al.,* 2006). The efficacy of the silencing was demonstrated in BAC-GLT1-eGFP mice. To exclude potential non-specific effects of the LV due to the surgical procedure, injection of viral particles and potential impact on GLT1 promoter activity (Place *et al.,* 2008), they used BAC-GLAST-DsRed mice and they shown that the silencing was specific. Recently, Liu and colleagues have used a miR30-based gene driven by the GFA-ABC1D promoter to express siRNA (Liu *et al.,* 2010). However, in this study, the tropism of the vector was not assessed in vivo. The proof-of-principle study was limited to luciferase measurement in the brain, whether this signal was exclusively due to transgene expression in astrocytes was not investigated. Furthermore, they perform triple injections of LVs which can lead to an astrogliosis, modifying the natural tropism of LVs.

In the last part of study, the inventors have re-investigated the potential of VSV-G pseudotyping LVs for astrocytic targeting. VSV-G envelope is of particular interest because viral vectors are easier to produce and have higher viral titers than with MOK-G envelope (Colin *et al.,* 2009; Desmaris *et al.,* 2001; Liu *et al.,* 2008). Here, they confirmed the promoter-dependence on the tropism of VSV-G pseudotyped LV (Jakobsson *et al.,* 2003; Liu *et al.,* 2010; Meunier *et al.,* 2008; Miletic *et al.,* 2004). The receptor for VSV-G is still unknown: phosphatidylserine has first been proposed (Schlegel *et al.,* 1983), but following studies suggest that it is enhancing viral entry and is not the receptor for the VSV-G envelope (Coil and Miller, 2004, 2005). Recently, it has been shown that the endoplasmic reticulum chaperone gp96, ubiquitously expressed, is essential for the occurrence of functional VSV-G receptors at the cell surface (Bloor *et al.,* 2010). This finding provides evidence for a broad host range of VSV-G pseudotyped LVs including neurons and astrocytes. Hence, tropism of VSV-G pseudotyped LVs is the result of the combined effect of the envelope and the promoter. By combining the VSV-G pseudotyping with GS promoter, miR124T, and an inducible system, the inventors finally show that an astrocytic silencing is efficiently achieved.

These vectors would be particularly suitable for HD study. Indeed, GS promoter activity is not affected by the expression of mHtt. The protein level is unaltered in HD patients (Carter, 1982) and in R6/2 HD transgenic mice (Behrens *et al.,* 2002).

### REFERENCES

Adra, C.N., Boer, P.H., and McBurney, M.W. (1987). Cloning and expression of the mouse pgk-1 gene and the nucleotide sequence of its promoter. Gene 60, 65-74.
Behrens, P.F., Franz, P., Woodman, B., Lindenberg, K.S., and Landwehrmeyer, G.B. (2002). Impaired glutamate transport and glutamate-glutamine cycling: downstream effects of the Huntington mutation. Brain 125, 1908-1922.
Berger, U.V., and Hediger, M.A. (1998). Comparative analysis of glutamate transporter expression in rat brain using differential double in situ hybridization. Anat Embryol (Berl) 198, 13-30.
Blesch, A., Conner, J., Pfeifer, A., Gasmi, M., Ramirez, A., Britton, W., Alfa, R., Verma, I., and Tuszynski, M.H. (2005). Regulated lentiviral NGF gene transfer controls rescue of medial septal cholinergic neurons. Mol Ther 11, 916-925.
Bloor, S., Maelfait, J., Krumbach, R., Beyaert, R., and Randow, F. (2010). Endoplasmic reticulum chaperone gp96 is essential for infection with vesicular stomatitis virus. Proc Natl Acad Sci U S A 107, 6970-6975.
Boudreau, R.L., Martins, I., and Davidson, B.L. (2009). Artificial microRNAs as siRNA shuttles: improved safety as compared to shRNAs in vitro and in vivo. Mol Ther 17, 169-175.
Boudreau, R.L., Monteys, A.M., and Davidson, B.L. (2008). Minimizing variables among hairpin-based RNAi vectors reveals the potency of shRNAs. RNA 14, 1834-1844.
Bradford, J., Shin, J.Y., Roberts, M., Wang, C.E., Li, X.J., and Li, S. (2009). Expression of mutant huntingtin in mouse brain astrocytes causes age-dependent neurological symptoms. Proc Natl Acad Sci U S A 106, 22480-22485.
Bradford, J., Shin, J.Y., Roberts, M., Wang, C.E., Sheng, G., Li, S., and Li, X.J. (2010). Mutant huntingtin in glial cells exacerbates neurological symptoms of Huntington disease mice. J Biol Chem 285, 10653-10661.
Brown, B.D., Cantore, A., Annoni, A., Sergi, L.S., Lombardo, A., Della Valle, P., D'Angelo, A., and Naldini, L. (2007a). A microRNA-regulated lentiviral vector mediates stable correction of hemophilia B mice. Blood 110, 4144-4152.
Brown, B.D., Gentner, B., Cantore, A., Colleoni, S., Amendola, M., Zingale, A., Baccarini, A., Lazzari, G., Gaili, C., and Naldini, L. (2007b). Endogenous microRNA can be broadly exploited to regulate transgene expression according to tissue, lineage and differentiation state. Nat Biotechnol 25, 1457-1467.
Brown, B.D., Venneri, M.A., Zingale, A., Sergi Sergi, L., and Naldini, L. (2006). Endogenous microRNA regulation suppresses transgene expression in hematopoietic lineages and enables stable gene transfer. Nat Med 12, 585-591.
Cannon, J.R., Sew, T., Montero, L., Burton, E.A., and Greenamyre, J.T. (2010). Pseudotype-dependent lentiviral transduction of astrocytes or neurons in the rat substantia nigra. Exp Neurol. Carter, C.J. (1982). Glutamine synthetase activity in Huntington's disease. Life Sci 31, 1151-1159.
Chaudhry, F.A., Lehre, K.P., van Lookeren Campagne, M., Ottersen, O.P., Danbolt, N.C., and Storm-Mathisen, J. (1995). Glutamate transporters in glial plasma membranes: highly differentiated localizations revealed by quantitative ultrastructural immunocytochemistry. Neuron 15, 711-720.
Chen, W., Aoki, C., Mahadomrongkul, V., Gruber, C.E., Wang, G.J., Blitzblau, R., Irwin, N., and Rosenberg, P.A. (2002). Expression of a variant form of the glutamate transporter GLT1 in neuronal cultures and in neurons and astrocytes in the rat brain. J Neurosci 22, 2142-2152.
Coil, D.A., and Miller, A.D. (2004). Phosphatidylserine is not the cell surface receptor for vesicular stomatitis virus. J Virol 78, 10920-10926.
Coil, D.A., and Miller, A.D. (2005). Enhancement of enveloped virus entry by phosphatidylserine. J Virol 79, 11496-11500.
Colin, A., Faideau, M., Dufour, N., Auregan, G., Hassig, R., Andrieu, T., Brouillet, E., Hantraye, P., Bonvento, G., and Deglon, N. (2009). Engineered lentiviral vector targeting astrocytes in vivo. Glia 57, 667-679.
D'Souza, C.A., Chopra, V., Varhol, R., Xie, Y.Y., Bohacec, S., Zhao, Y., Lee, L.L., Bilenky, M., Portales-Casamar, E., He, A., et al. (2008). Identification of a set of genes showing regionally enriched expression in the mouse brain. BMC Neurosci 9, 66.
de Vivo, L., Melone, M., Rothstein, J.D., and Conti, F. (2010). GLT-1 Promoter Activity in Astrocytes and Neurons of Mouse Hippocampus and Somatic Sensory Cortex. Front Neuroanat 3, 31.
Deglon, N., Tseng, J.L., Bensadoun, J.C., Zurn, A.D., Arsenijevic, Y., Pereira de Almeida, L., Zufferey, R., Trono, D., and Aebischer, P. (2000). Self-inactivating lentiviral vectors with enhanced transgene expression as potential gene transfer system in Parkinson's disease. Hum Gene Ther 11, 179-190.
Deo, M., Yu, J.Y., Chung, K.H., Tippens, M., and Turner, D.L. (2006). Detection of mammalian microRNA expression by in situ hybridization with RNA oligonucleotides. Dev Dyn 235, 2538-2548. Desmaris, N., Bosch, A., Salaun, C., Petit, C., Prevost, M.C., Tordo, N., Perrin, P., Schwartz, O., de Rocquigny, H., and Heard, J.M. (2001). Production and neurotropism of lentivirus vectors pseudotyped with lyssavirus envelope glycoproteins. Mol Ther 4, 149-156.
Doench, J.G., Petersen, C.P., and Sharp, P.A. (2003). siRNAs can function as miRNAs. Genes Dev 17, 438-442.
ENCODE (2004). The ENCODE (ENCyclopedia Of DNA Elements) Project. Science 306, 636-640.
Faideau, M., Kim, J., Cormier, K., Gilmore, R., Welch, M., Auregan, G., Dufour, N., Guillermier, M., Brouillet, E., Hantraye, P., et al. (2010). In vivo expression of polyglutamine-expanded huntingtin by mouse striatal astrocytes impairs glutamate transport: a correlation with Huntington's disease subjects. Hum Mol Genet.
Franklin, K., and Paxinos, G. (1997). The Mouse Brain in Stereotaxic Coordinates. San Diego: Academic Press.
Furuta, A., Rothstein, J.D., and Martin, L.J. (1997). Glutamate transporter protein subtypes are expressed differentially during rat CNS development. J Neurosci 17, 8363-8375.
Han, L., Wong, D.L., Tsai, G., Jiang, Z., and Coyle, J.T. (2007). Promoter analysis of human glutamate carboxypeptidase II. Brain Res 1170, 1-12.
Haydon, P.G., and Carmignoto, G. (2006). Astrocyte control of synaptic transmission and neurovascular coupling. Physiol Rev 86, 1009-1031.
Hirrlinger, P.G., Scheller, A., Braun, C., Hirrlinger, J., and Kirchhoff, F. (2006). Temporal control of gene recombination in astrocytes by transgenic expression of the tamoxifen-inducible DNA recombinase variant CreERT2. Glia 54, 11-20.
Hottinger, A.F., Azzouz, M., Deglon, N., Aebischer, P., and Zurn, A.D. (2000). Complete and long-term rescue of lesioned adult motoneurons by lentiviral-mediated expression of glial cell line-derived neurotrophic factor in the facial nucleus. J Neurosci 20, 5587-5593.
Jakobsson, J., Ericson, C., Jansson, M., Bjork, E., and Lundberg, C. (2003). Targeted transgene expression in rat brain using lentiviral vectors. J Neurosci Res 73, 876-885.
Jakobsson, J., and Lundberg, C. (2006). Lentiviral vectors for use in the central nervous system. Mol Ther 13, 484-493.
Kim, S.Y., Choi, S.Y., Chao, W., and Volsky, D.J. (2003). Transcriptional regulation of human excitatory amino acid transporter 1 (EAAT1): cloning of the EAAT1 promoter and characterization of its basal and inducible activity in human astrocytes. J Neurochem 87, 1485-1498.
Krebs, C.J., McAvoy, M.T., and Kumar, G. (1995). The JC virus minimal core promoter is glial cell specific in vivo. J Virol 69, 2434-2442.
Kugler, S., Meyn, L., Holzmuller, H., Gerhardt, E., Isenmann, S., Schulz, J.B., and Bahr, M. (2001). Neuron-specific expression of therapeutic proteins: evaluation of different cellular promoters in recombinant adenoviral vectors. Mol Cell Neurosci 17, 78-96.
Kuroda, H., Kutner, R.H., Bazan, N.G., and Reiser, J. (2008). A comparative analysis of constitutive and cell-specific promoters in the adult mouse hippocampus using lentivirus vector-mediated gene transfer. J Gene Med 10, 1163-1175.
Lagos-Quintana, M., Rauhut, R., Yalcin, A., Meyer, J., Lendeckel, W., and Tuschl, T. (2002). Identification of tissue-specific microRNAs from mouse. Curr Biol 12, 735-739.
Lee, Y., Messing, A., Su, M., and Brenner, M. (2008). GFAP promoter elements required for region-specific and astrocyte-specific expression. Glia 56, 481-493.
Lehre, K.P., Levy, L.M., Ottersen, O.P., Storm-Mathisen, J., and Danbolt, N.C. (1995). Differential expression of two glial glutamate transporters in the rat brain: quantitative and immunocytochemical observations. J Neurosci 15, 1835-1853.
Li, M., Husic, N., Lin, Y., Christensen, H., Malik, I., McIver, S., Daniels, C.M., Harris, D.A., Kotzbauer, P.T., Goldberg, M.P., et al. (2010). Optimal promoter usage for lentiviral vector-mediated transduction of cultured central nervous system cells. J Neurosci Methods 189, 56-64.
Liu, B., Paton, J.F., and Kasparov, S. (2008). Viral vectors based on bidirectional cell-specific mammalian promoters and transcriptional amplification strategy for use in vitro and in vivo. BMC Biotechnol 8, 49.
Liu, B., Xu, H., Paton, J.F., and Kasparov, S. (2010). Cell- and region-specific miR30-based gene knock-down with temporal control in the rat brain. BMC Mol Biol 11, 93.
Lobsiger, C.S., and Cleveland, D.W. (2007). Glial cells as intrinsic components of non-cell-autonomous neurodegenerative disease. Nat Neurosci 10, 1355-1360.
Maragakis, N.J., and Rothstein, J.D. (2006). Mechanisms of Disease: astrocytes in neurodegenerative disease. Nat Clin Pract Neurol 2, 679-689.
Martin, L.J., Brambrink, A.M., Lehmann, C., Portera-Cailliau, C., Koehler, R., Rothstein, J., and Traystman, R.J. (1997). Hypoxia-ischemia causes abnormalities in glutamate transporters and death of astroglia and neurons in newborn striatum. Ann Neurol 42, 335-348.
McBride, J.L., Boudreau, R.L., Harper, S.Q., Staber, P.D., Monteys, A.M., Martins, I., Gilmore, B.L., Burstein, H., Peluso, R.W., Polisky, B., et al. (2008). Artificial miRNAs mitigate shRNA-mediated toxicity in the brain: implications for the therapeutic development of RNAi. Proc Natl Acad Sci U S A 105, 5868-5873.
Mearow, K.M., Mill, J.F., and Vitkovic, L. (1989). The ontogeny and localization of glutamine synthetase gene expression in rat brain. Brain Res Mol Brain Res 6, 223-232.
Mennerick, S., Dhond, R.P., Benz, A., Xu, W., Rothstein, J.D., Danbolt, N.C., Isenberg, K.E., and Zorumski, C.F. (1998). Neuronal expression of the glutamate transporter GLT-1 in hippocampal microcultures. J Neurosci 18, 4490-4499.
Meunier, A., Mauborgne, A., Masson, J., Mallet, J., and Pohl, M. (2008). Lentiviral-mediated targeted transgene expression in dorsal spinal cord glia: tool for the study of glial cell implication in mechanisms underlying chronic pain development. J Neurosci Methods 167, 148-159.
Miletic, H., Fischer, Y.H., Neumann, H., Hans, V., Stenzel, W., Giroglou, T., Hermann, M., Deckert, M., and Von Laer, D. (2004). Selective transduction of malignant glioma by lentiviral vectors pseudotyped with lymphocytic choriomeningitis virus glycoproteins. Hum Gene Ther 15, 1091-1100. Mill, J.F., Mearow, K.M., Purohit, H.J., Haleem-Smith, H., King, R., and Freese, E. (1991). Cloning and functional characterization of the rat glutamine synthetase gene. Brain Res Mol Brain Res 9, 197-207.
Mishima, T., Mizuguchi, Y., Kawahigashi, Y., and Takizawa, T. (2007). RT-PCR-based analysis of microRNA (miR-1 and -124) expression in mouse CNS. Brain Res 1131, 37-43.
Mori, T., Tanaka, K., Buffo, A., Wurst, W., Kuhn, R., and Gotz, M. (2006). Inducible gene deletion in astroglia and radial glia--a valuable tool for functional and lineage analysis. Glia 54, 21-34.
Naldini, L., Blomer, U., Gallay, P., Ory, D., Mulligan, R., Gage, F.H., Verma, I.M., and Trono, D. (1996). In vivo gene delivery and stable transduction of nondividing cells by a lentiviral vector. Science 272, 263-267.
Northington, F.J., Traystman, R.J., Koehler, R.C., and Martin, L.J. (1999). GLT1, glial glutamate transporter, is transiently expressed in neurons and develops astrocyte specificity only after midgestation in the ovine fetal brain. J Neurobiol 39, 515-526.
Pertusa, M., Garcia-Matas, S., Mammeri, H., Adell, A., Rodrigo, T., Mallet, J., Cristofol, R., Sarkis, C., and Sanfeliu, C. (2008). Expression of GDNF transgene in astrocytes improves cognitive deficits in aged rats. Neurobiol Aging 29, 1366-1379.
Place, R.F., Li, L.C., Pookot, D., Noonan, E.J., and Dahiya, R. (2008). MicroRNA-373 induces expression of genes with complementary promoter sequences. Proc Natl Acad Sci U S A 105, 1608-1613.
Portales-Casamar, E., Swanson, D.J., Liu, L., de Leeuw, C.N., Banks, K.G., Ho Sui, S.J., Fulton, D.L., Ali, J., Amirabbasi, M., Arenillas, D.J., et al. (2010). A regulatory toolbox of MiniPromoters to drive selective expression in the brain. Proc Natl Acad Sci U S A 107, 16589-16594.
Powers, W.J., Videen, T.O., Markham, J., McGee-Minnich, L., Antenor-Dorsey, J.V., Hershey, T., and Perlmutter, J.S. (2007). Selective defect of in vivo glycolysis in early Huntington's disease striatum. Proc Natl Acad Sci U S A 104, 2945-2949.
Regan, M.R., Huang, Y.H., Kim, Y.S., Dykes-Hoberg, M.I., Jin, L., Watkins, A.M., Bergles, D.E., and Rothstein, J.D. (2007). Variations in promoter activity reveal a differential expression and physiology of glutamate transporters by glia in the developing and mature CNS. J Neurosci 27, 6607-6619.
Regulier, E., Pereira de Almeida, L., Sommer, B., Aebischer, P., and Deglon, N. (2002). Dose-dependent neuroprotective effect of ciliary neurotrophic factor delivered via tetracycline-regulated lentiviral vectors in the quinolinic acid rat model of Huntington's disease. Hum Gene Ther 13, 1981-1990.
Rothstein, J.D., Martin, L., Levey, A.I., Dykes-Hoberg, M., Jin, L., Wu, D., Nash, N., and Kuncl, R.W. (1994). Localization of neuronal and glial glutamate transporters. Neuron 13, 713-725.
Sarkis, C., Serguera, C., Petres, S., Buchet, D., Ridet, J.L., Edelman, L., and Mallet, J. (2000). Efficient transduction of neural cells in vitro and in vivo by a baculovirus-derived vector. Proc Natl Acad Sci U S A 97, 14638-14643.
Schlegel, R., Tralka, T.S., Willingham, M.C., and Pastan, I. (1983). Inhibition of VSV binding and infectivity by phosphatidylserine: is phosphatidylserine a VSV-binding site? Cell 32, 639-646.
Shin, J.Y., Fang, Z.H., Yu, Z.X., Wang, C.E., Li, S.H., and Li, X.J. (2005). Expression of mutant huntingtin in glial cells contributes to neuronal excitotoxicity. J Cell Biol 171, 1001-1012.
Shin, K.J., Wall, E.A., Zavzavadjian, J.R., Santat, L.A., Liu, J., Hwang, J.I., Rebres, R., Roach, T., Seaman, W., Simon, M.I., et al. (2006). A single lentiviral vector platform for microRNA-based conditional RNA interference and coordinated transgene expression. Proc Natl Acad Sci U S A 103, 13759-13764.
Slezak, M., Goritz, C., Niemiec, A., Frisen, J., Chambon, P., Metzger, D., and Pfrieger, F.W. (2007). Transgenic mice for conditional gene manipulation in astroglial cells. Glia 55, 1565-1576.
Slezak, M., and Pfrieger, F.W. (2003). New roles for astrocytes: regulation of CNS synaptogenesis. Trends Neurosci 26, 531-535.
Smirnova, L., Grafe, A., Seiler, A., Schumacher, S., Nitsch, R., and Wulczyn, F.G. (2005). Regulation of miRNA expression during neural cell specification. Eur J Neurosci 21, 1469-1477.
Smith, B., Treadwell, J., Zhang, D., Ly, D., McKinnell, I., Walker, P.R., and Sikorska, M. (2010). Large-scale expression analysis reveals distinct microRNA profiles at different stages of human neurodevelopment. PLoS One 5, e11109.
Takano, T., Tian, G.F., Peng, W., Lou, N., Libionka, W., Han, X., and Nedergaard, M. (2006). Astrocyte-mediated control of cerebral blood flow. Nat Neurosci 9, 260-267.
Torp, R., Danbolt, N.C., Babaie, E., Bjoras, M., Seeberg, E., Storm-Mathisen, J., and Ottersen, O.P. (1994). Differential expression of two glial glutamate transporters in the rat brain: an in situ hybridization study. Eur J Neurosci 6, 936-942.
Vigna, E., Amendola, M., Benedicenti, F., Simmons, A.D., Follenzi, A., and Naldini, L. (2005). Efficient Tet-dependent expression of human factor IX in vivo by a new self-regulating lentiviral vector. Mol Ther 11, 763-775.
Volterra, A., and Meldolesi, J. (2005). Astrocytes, from brain glue to communication elements: the revolution continues. Nat Rev Neurosci 6, 626-640.
Voutsinos-Porche, B., Bonvento, G., Tanaka, K., Steiner, P., Welker, E., Chatton, J.Y., Magistretti, P.J., and Pellerin, L. (2003). Glial glutamate transporters mediate a functional metabolic crosstalk between neurons and astrocytes in the mouse developing cortex. Neuron 37, 275-286.
Watson, D.J., Kobinger, G.P., Passini, M.A., Wilson, J.M., and Wolfe, J.H. (2002). Targeted transduction patterns in the mouse brain by lentivirus vectors pseudotyped with VSV, Ebola, Mokola, LCMV, or MuLV envelope proteins. Mol Ther 5, 528-537.
Wong, L.F., Goodhead, L., Prat, C., Mitrophanous, K.A., Kingsman, S.M., and Mazarakis, N.D. (2006). Lentivirus-mediated gene transfer to the central nervous system: therapeutic and research applications. Hum Gene Ther 17, 1-9.
Yang, G.S., Banks, K.G., Bonaguro, R.J., Wilson, G., Dreolini, L., de Leeuw, C.N., Liu, L., Swanson, D.J., Goldowitz, D., Holt, R.A., et al. (2009). Next generation tools for high-throughput promoter and expression analysis employing single-copy knock-ins at the Hprt1 locus. Genomics 93, 196-204.
Yang, Y., Vidensky, S., Jin, L., Jie, C., Lorenzini, I., Frankl, M., and Rothstein, J.D. (2011). Molecular comparison of GLT1+ and ALDH1L1+ astrocytes in vivo in astroglial reporter mice. Glia 59, 200-207.

## Claims

1. A viral vector for silencing a gene specifically in astrocytes comprising:
- an astrocyte-specific viral envelope protein,
- a first nucleic acid sequence encoding a transcription activator and at least one target sequence of a neuron-specific miR under the control of an astrocyte-specific promoter, and
- a second nucleic acid sequence encoding a RNA for silencing the gene under the control of a promoter inducible by the transcription activator.

2. The viral vector according to claim 1, wherein the astrocyte-specific viral envelope protein is Mokola virus G protein (G-MOK).

3. The viral vector according to claim 1 or 2, wherein the transcription activator is the tetracycline transactivator (tTA) and the promoter inducible by the transcription activator is the tetracycline response element (TRE).

4. The viral vector according to any of claims 1 to 3, wherein the astrocyte specific promoter is the promoter of the glutamine synthase (GS) gene, preferably the promoter of rat GS gene.

5. The viral vector according to any of claims 1 to 4, wherein the target sequence of the neuron-specific miR is a target sequence of miR124 (miR124T).

6. The viral vector according to any of claims 1 to 5, wherein the nucleic acid sequence encoding a RNA for silencing the gene encodes a siRNA or a shRNA targeting the gene mRNA.

7. The viral vector according to claim 6, wherein the shRNA is embedded in a miR sequence, preferably a miR30 sequence.

8. The viral vector according to any of claims 1 to 7, wherein the viral vector is a lentiviral vector, preferably a HIV-1 vector.

9. The viral vector according to any of claims 1 to 8, wherein the first and the second nucleic acid sequences are on a same nucleic acid molecule.

10. The viral vector according to claim 9, wherein the nucleic acid molecule comprises, from 5' to 3':
- a 5' long terminal repeat (LTR) sequence,
- a sequence encoding a shRNA targeting the gene embedded in a miR30 sequence,
- the GS rat promoter,
- a sequence encoding the tetracycline transactivator
- the woodchuck hepatitis post-transcriptional regulatory element (WPRE)
- a sequence encoding 4 copies of a miR124 target sequence,
- a 3' long terminal repeat (LTR) sequence comprising the tetracycline response element (TRE).

11. The viral vector according to any of claims 1 to 10, wherein the gene is the huntingtin gene.

12. The viral vector according to any of claims 1 to 11, for use in the prevention or treatment of astrocyte-mediated diseases.

13. A pharmaceutical composition comprising a viral vector according to any of claims 1 to 11 as an active ingredient, optionally in association with a pharmaceutically acceptable vehicle or excipient.

14. A method, in particular an in vitro method, for silencing a gene in astrocytes, comprising contacting a viral vector according to any of claims 1 to 11 with at least one astrocyte.

15. A nucleic acid molecule comprising from 5' to 3':
- a 5' long terminal repeat (LTR) sequence,
- a sequence comprising from 4 to 1000 nucleotides,
- the GS rat promoter,
- a sequence encoding the tetracycline transactivator,
- the woodchuck hepatitis post-transcriptional regulatory element (WPRE),
- a sequence encoding 4 copies of a miR124 target sequence,
- a 3' long terminal repeat (LTR) sequence comprising the tetracycline response element (TRE).

16. The nucleic acid sequence according to claim 15, wherein the sequence comprising from 4 to 1000 nucleotides is a multiple cloning site.

17. The nucleic acid sequence according to claim 15, wherein the sequence comprising from 4 to 1000 nucleotides is sequence encoding a siRNA or a shRNA.


SEQUENCE LISTING

<110>  COMMISSARIAT A L'ENERGIE ATOMIQUE ET AUX ENERGIES
       ALTERNATIVES

<120>  VECTOR FOR THE SELECTIVE SILENCING OF A GENE IN ASTROCYTES

<130>  F263-493

<160>  14

<170>  PatentIn version 3.5

<210>  1
<211>  747
<212>  DNA
<213>  Artificial

<220>
<223>  Tetracycline transactivator coding sequence

<400>  1
atgtctagac tggacaagag caaagtcata aactctgctc tggaattact caatgaagtc      60

ggtatcgaag gcctgacgac aaggaaactc gctcaaaagc tgggagttga gcagcctacc     120

ctgtactggc acgtgaagaa caagcgggcc ctgctcgatg ccctggcaat cgagatgctg     180

gacaggcatc atacccactt ctgccccctg gaaggcgagt catggcaaga ctttctgcgg     240

aacaacgcca agtcattccg ctgtgctctc ctctcacatc gcgacggggc taaagtgcat     300

ctcggcaccc gcccaacaga gaaacagtac gaaaccctgg aaaatcagct cgcgttcctg     360

tgtcagcaag gcttctccct ggagaacgca ctgtacgctc tgtccgccgt gggccacttt     420

acactgggct gcgtattgga ggatcaggag catcaagtag caaaagagga aagagagaca     480

cctaccaccg attctatgcc cccacttctg agacaagcaa ttgagctgtt cgaccatcag     540

ggagccgaac ctgccttcct tttcggcctg gaactaatca tatgtggcct ggagaaacag     600

ctaaagtgcg aaagcggcgg gccggccgac gcccttgacg attttgactt agacatgctc     660

ccagccgatg cccttgacga ctttgacctt gatatgctgc ctgctgacgc tcttgacgat     720

tttgaccttg acatgctccc cgggtaa                                         747

<210>  2
<211>  277
<212>  DNA
<213>  Artificial

<220>
<223>  Tetracycline response element

<400>  2
tcgagtttac tccctatcag tgatagagaa cgtatgtcga gtttactccc tatcagtgat      60

agagaacgat gtcgagttta ctccctatca gtgatagaga acgtatgtcg agtttactcc     120

ctatcagtga tagagaacgt atgtcgagtt tactccctat cagtgataga gaacgtatgt     180

cgagtttatc cctatcagtg atagagaacg tatgtcgagt ttactcccta tcagtgatag     240

agaacgtatg tcgaggtagg cgtgtacggt gggaggc 277

<210> 3
<211> 433
<212> DNA
<213> Artificial

<220>
<223> Rat glutamine synthase promoter

<400> 3
tggctcgctc aacaaagggt aaaattctct tctggggttc tggattttca ccgacagatc 60

tctccgaaaa cctctgtact gttaaggtag caaaggacca aggccaaatg acgctgagat 120

agtctcagcc tgggtccgag ggtccagagg tcgctaaaga acgcgtgctg caaaggcgca 180

cagcaccttc ggccccggcc ccccagccgc cgctgcctcc cctcccccat tcccctttct 240

ctttctttct tctgggtcgc tcgccccacc ccgcggttcc tcacggctcc tgggccaatg 300

gtctcagggc cccggtgcac agcaactgat gggcacgggg ttccaggcct aggccagcca 360

atcagggcgc ctgggggcgg gcacaagctg ccaataaaaa gtactgagca gcccgcaacc 420

ctccacagcc gag 433

<210> 4
<211> 21
<212> DNA
<213> Artificial

<220>
<223> miR124 target coding sequence

<400> 4
taaggcacgc ggtgaatgcc a 21

<210> 5
<211> 860
<212> DNA
<213> Artificial sequence

<220>
<223> Chimeric 5′ LTR sequence

<400> 5
aagcttggcc attgcatacg ttgtatccat atcataatat gtacatttat attggctcat 60

gtccaacatt accgccatgt tgacattgat tattgactag ttattaatag taatcaatta 120

cggggtcatt agttcatagc ccatatatgg agttccgcgt tacataactt acggtaaatg 180

gcccgcctgg ctgaccgccc aacgaccccc gcccattgac gtcaataatg acgtatgttc 240

ccatagtaac gccaataggg actttccatt gacgtcaatg ggtggagtat ttacggtaaa 300

ctgcccactt ggcagtacat caagtgtatc atatgccaag tacgccccct attgacgtca 360

atgacggtaa atggcccgcc tggcattatg cccagtacat gaccttatgg gactttccta 420

cttggcagta catctacgta ttagtcatcg ctattaccat ggtgatgcgg ttttggcagt 480

acatcaatgg gcgtggatag cggtttgact cacggggatt tccaagtctc caccccattg 540

acgtcaatgg gagtttgttt tggcaccaaa atcaacggga ctttccaaaa tgtcgtaaca 600

actccgcccc attgacgcaa atgggcggta ggcgtgtacg gtgggaggtc tatataagca 660

gagctcgttt agtgaaccgg ggtctctctg gttagaccag atctgagcct gggagctctc 720

tggctaacta gggaacccac tgcttaagcc tcaataaagc ttgccttgag tgcttcaagt 780

agtgtgtgcc cgtctgttgt gtgactctgg taactagaga tccctcagac ccttttagtc 840

agtgtggaaa atctctagca 860

<210> 6
<211> 610
<212> DNA
<213> Artificial

<220>
<223> Woodchuck hepatitis post-transcriptional regulatory element

<400> 6
gaattccgat aatcaacctc tggattacaa aatttgtgaa agattgactg gtattcttaa 60

ctatgttgct ccttttacgc tatgtggata cgctgcttta atgcctttgt atcatgctat 120

tgcttcccgt atggctttca ttttctcctc cttgtataaa tcctggttgc tgtctcttta 180

tgaggagttg tggcccgttg tcaggcaacg tggcgtggtg tgcactgtgt ttgctgacgc 240

aacccccact ggttggggca ttgccaccac ctgtcagctc ctttccggga ctttcgcttt 300

ccccctccct attgccacgg cggaactcat cgccgcctgc cttgcccgct gctggacagg 360

ggctcggctg ttgggcactg acaattccgt ggtgttgtcg gggaagctga cgtcctttcc 420

atggctgctc gcctgtgttg ccacctggat tctgcgcggg acgtccttct gctacgtccc 480

ttcggccctc aatccagcgg accttccttc ccgcggcctg ctgccggctc tgcggcctct 540

tccgcgtctt cgccttcgcc ctcagacgag tcggatctcc ctttgggccg cctccccgca 600

tcgggaattc 610

<210> 7
<211> 105
<212> DNA
<213> Artificial

<220>
<223> Sequence encoding 4 copies of miR124T

<400> 7
taaggcacgc ggtgaatgcc aatgcattta aggcacgcgg tgaatgccaa cgcgtttaag 60

gcacgcggtg aatgccattc gaattaaggc acgcggtgaa tgcca 105

<210> 8
<211> 594

<212>  DNA
<213>  Artificial

<220>
<223>  Self-inactivating 3'LTR comprising the CMV promoter

<400>  8
tggaagggct aattcactcc caacgaagac aagatatgat cccgggctcg agtttaccac      60

tccctatcag tgatagagaa aagtgaaagt cgagtttacc actccctatc agtgatagag     120

aaaagtgaaa gtcgagttta ccactcccta tcagtgatag agaaaagtga aagtcgagtt     180

taccactccc tatcagtgat agagaaaagt gaaagtcgag tttaccactc cctatcagtg     240

atagagaaaa gtgaaagtcg agtttaccac tccctatcag tgatagagaa aagtgaaagt     300

cgagtttacc actccctatc agtgatagag aaaagtgaaa gtcgagctcg gtacccgggt     360

cgagtaggcg ttgatcagat gctgcatata agcagctgct ttttgcttgt actgggtctc     420

tctggttaga ccagatctga gcctgggagc tctctggcta actagggaac ccactgctta     480

agcctcaata aagcttgcct tgagtgcttc aagtagtgtg tgcccgtctg ttgtgtgact     540

ctggtaacta gagatccctc agaccctttt agtcagtgtg gaaaatctct agca           594


<210>  9
<211>  64
<212>  DNA
<213>  Artificial

<220>
<223>  Anti-huntingtin shRNA coding sequence

<400>  9
agcgaagctt tgatggattc taattagtga agccacagat gtaattagaa tccatcaaag      60

ctct                                                                   64


<210>  10
<211>  340
<212>  DNA
<213>  Artificial

<220>
<223>  miR30-embedded anti-huntingtin shRNA coding sequence

<400>  10
ggatccgtcg actagggata acagggtaat tgtttgaatg aggcttcagt actttacaga      60

atcgttgcct gcacatcttg gaaacacttg ctgggattac ttcttcaggt taacccaaca     120

gaaggctcga gaaggtatat tgctgttgac agtgagcgaa gctttgatgg attctaatta     180

gtgaagccac agatgtaatt agaatccatc aaagctctgc ctactgcctc ggaattcaag     240

gggctacttt aggagcaatt atcttgttta ctaaaactga ataccttgct atctctttga     300

tacattttta caaagctgaa ttaaaatggt ataaattaaa                           340


<210>  11

<211>  5457
<212>  DNA
<213>  Artificial

<220>
<223>  Construct for inactivating the huntigtin gene specifically in
       astrocytes

<400>  11
aagcttggcc attgcatacg ttgtatccat atcataatat gtacatttat attggctcat      60

gtccaacatt accgccatgt tgacattgat tattgactag ttattaatag taatcaatta     120

cggggtcatt agttcatagc ccatatatgg agttccgcgt tacataactt acggtaaatg     180

gcccgcctgg ctgaccgccc aacgaccccc gcccattgac gtcaataatg acgtatgttc     240

ccatagtaac gccaataggg actttccatt gacgtcaatg ggtggagtat ttacggtaaa     300

ctgcccactt ggcagtacat caagtgtatc atatgccaag tacgccccct attgacgtca     360

atgacggtaa atggcccgcc tggcattatg cccagtacat gaccttatgg gactttccta     420

cttggcagta catctacgta ttagtcatcg ctattaccat ggtgatgcgg ttttggcagt     480

acatcaatgg gcgtggatag cggtttgact cacggggatt tccaagtctc caccccattg     540

acgtcaatgg gagtttgttt tggcaccaaa atcaacggga ctttccaaaa tgtcgtaaca     600

actccgcccc attgacgcaa atgggcggta ggcgtgtacg gtgggaggtc tatataagca     660

gagctcgttt agtgaaccgg ggtctctctg gttagaccag atctgagcct gggagctctc     720

tggctaacta gggaacccac tgcttaagcc tcaataaagc ttgccttgag tgcttcaagt     780

agtgtgtgcc cgtctgttgt gtgactctgg taactagaga tccctcagac ccttttagtc     840

agtgtggaaa atctctagca gtggcgcccg aacagggacc tgaaagcgaa agggaaacca     900

gagctctctc gacgcaggac tcggcttgct gaagcgcgca cggcaagagg cgaggggcgg     960

cgactggtga gtacgccaaa aattttgact agcggaggct agaaggagag agatgggtgc    1020

gagagcgtca gtattaagcg ggggagaatt agatcgcgat gggaaaaaat tcggttaagg    1080

ccagggggaa agaaaaaata taaattaaaa catatagtat gggcaagcag ggagctagaa    1140

cgattcgcag ttaatcctgg cctgttagaa acatcagaag gctgtagaca aatactggga    1200

cagctacaac catcccttca gacaggatca gaagaactta gatcattata taatacagta    1260

gcaaccctct attgtgtgca tcaaaggata gagataaaag acaccaagga agctttagac    1320

aagatagagg aagagcaaaa caaaagtaag accaccgcac agcaagcggc cgctgatctt    1380

cagacctgga ggaggagata tgagggacaa ttggagaagt gaattatata aatataaagt    1440

agtaaaaatt gaaccattag gagtagcacc caccaaggca aagagaagag tggtgcagag    1500

agaaaaaaga gcagtgggaa taggagcttt gttccttggg ttcttgggag cagcaggaag    1560

cactatgggc gcagcctcaa tgacgctgac ggtacaggcc agacaattat tgtctggtat    1620

agtgcagcag cagaacaatt tgctgagggc tattgaggcg caacagcatc tgttgcaact    1680

cacagtctgg ggcatcaagc agctccaggc aagaatcctg gctgtggaaa gatacctaaa 1740

ggatcaacag ctcctgggga tttggggttg ctctggaaaa ctcatttgca ccactgctgt 1800

gccttggaat gctagttgga gtaataaatc tctggaacag atttggaatc acacgacctg 1860

gatggagtgg gacagagaaa ttaacaatta cacaagctta atacactcct taattgaaga 1920

atcgcaaaac cagcaagaaa agaatgaaca agaattattg gaattagata aatgggcaag 1980

tttgtggaat tggtttaaca taacaaattg gctgtggtat ataaaattat tcataatgat 2040

agtaggaggc ttggtaggtt taagaatagt ttttgctgta ctttctatag tgaatagagt 2100

taggcaggga tattcaccat tatcgtttca gacccacctc ccaaccccga ggggacccga 2160

caggcccgaa ggaatagaag aagaaggtgg agagagagac agagacagat ccattcgatt 2220

agtgaacgga tctcgacggt atcggttatc cagggatcca ccggtatcca tgctagcgga 2280

tccgtcgact agggataaca gggtaattgt ttgaatgagg cttcagtact ttacagaatc 2340

gttgcctgca catcttggaa acacttgctg ggattacttc ttcaggttaa cccaacagaa 2400

ggctcgagaa ggtatattgc tgttgacagt gagcgagcaa gctgaccctg aagttcatct 2460

gtgaagccac agatgggatg aacttcaggg tcagcttgcc tgcctactgc ctcggaattc 2520

aaggggctac tttaggagca attatcttgt ttactaaaac tgaatacctt gctatctctt 2580

tgatacattt ttacaaagct gaattaaaat ggtataaatt aaactcgagg ctagcctgca 2640

gacgcgtgtt aacttttaaa agaaaagggg ggattggggg gtacagtgca ggggaaagaa 2700

tagtagacat aatagcaaca gacatacaaa ctaaagaatt acaaaaacaa attacaaaaa 2760

ttcaaaattt tgtcgacaat caacctctgg attacaaaat ttgtgaaaga ttgactggta 2820

ttcttaacta tgttgctcct tttacgctat gtggatacgc tgctttaatg cctttgtatc 2880

atgctattgc ttcccgtatg gctttcattt tctcctcctt gtataaatcc tggttgctgt 2940

ctctttatga ggagttgtgg cccgttgtca ggcaacgtgg cgtggtgtgc actgtgtttg 3000

ctgacgcaac ccccactggt tggggcattg ccaccacctg tcagctcctt tccgggactt 3060

tcgctttccc cctccctatt gccacggcgg aactcatcgc cgcctgcctt gcccgctgct 3120

ggacaggggc tcggctgttg ggcactgaca attccgtggt gttgtcgggg aagctgacgt 3180

cctttccatg gctgctcgcc tgtgttgcca cctggattct gcgcgggacg tccttctgct 3240

acgtcccttc ggccctcaat ccagcggacc ttccttcccg cggcctgctg ccggctctgc 3300

ggcctcttcc gcgtcttcgc cttcgccctc agacgagtcg gatctccctt tgggccgcct 3360

ccccgcctgg gaattgatcc cgatggctcg ctcaacaaag ggtaaaattc tcttctgggg 3420

ttctggattt tcaccgacag atctctccga aaacctctgt actgttaagg tagcaaagga 3480

ccaaggccaa atgacgctga gatagtctca gcctgggtcc gagggtccag aggtcgctaa 3540

agaacgcgtg ctgcaaaggc gcacagcacc ttcggccccg gccccccagc cgccgctgcc 3600

tcccctcccc cattcccctt tctctttctt tcttctgggt cgctcgcccc accccgcggt 3660

tcctcacggc tcctgggcca atggtctcag ggccccggtg cacagcaact gatgggcacg 3720

gggttccagg cctaggccag ccaatcaggg cgcctggggg cgggcacaag ctgccaataa 3780

aaagtactga gcagcccgca accctccaca gccgagggat ccaagggtgg gcgcgccgac 3840

ccagctttct tgtacaaagt ggtgataatt cctgcagccc gggggatcca ctagttctag 3900

gatccaccat gtctagactg gacaagagca aagtcataaa ctctgctctg gaattactca 3960

atgaagtcgg tatcgaaggc ctgacgacaa ggaaactcgc tcaaaagctg ggagttgagc 4020

agcctaccct gtactggcac gtgaagaaca agcgggccct gctcgatgcc ctggcaatcg 4080

agatgctgga caggcatcat acccacttct gccccctgga aggcgagtca tggcaagact 4140

ttctgcggaa caacgccaag tcattccgct gtgctctcct ctcacatcgc gacggggcta 4200

aagtgcatct cggcacccgc ccaacagaga aacagtacga aaccctggaa aatcagctcg 4260

cgttcctgtg tcagcaaggc ttctccctgg agaacgcact gtacgctctg tccgccgtgg 4320

gccactttac actgggctgc gtattggagg atcaggagca tcaagtagca aaagaggaaa 4380

gagagacacc taccaccgat tctatgcccc cacttctgag acaagcaatt gagctgttcg 4440

accatcaggg agccgaacct gccttccttt tcggcctgga actaatcata tgtggcctgg 4500

agaaacagct aaagtgcgaa agcggcgggc cggccgacgc ccttgacgat tttgacttag 4560

acatgctccc agccgatgcc cttgacgact ttgaccttga tatgctgcct gctgacgctc 4620

ttgacgattt tgaccttgac atgctccccg ggtaactaag taaggatcga tatggcattc 4680

accgcgtgcc ttaattcgaa tggcattcac cgcgtgcctt aaacgcgttg gcattcaccg 4740

cgtgccttaa atgcattggc attcaccgcg tgccttactg cagggtacct taattccgtt 4800

taagaccaat gacttacaag gcagctgtag atcttagcca ctttttaaaa gaaaaggggg 4860

gactggaagg gctaattcac tcccaacgaa gacaagatat gatcccgggc tcgagtttac 4920

cactccctat cagtgataga gaaaagtgaa agtcgagttt accactccct atcagtgata 4980

gagaaaagtg aaagtcgagt ttaccactcc ctatcagtga tagagaaaag tgaaagtcga 5040

gtttaccact ccctatcagt gatagagaaa agtgaaagtc gagtttacca ctccctatca 5100

gtgatagaga aaagtgaaag tcgagtttac cactccctat cagtgataga gaaaagtgaa 5160

agtcgagttt accactccct atcagtgata gagaaaagtg aaagtcgagc tcggtacccg 5220

ggtcgagtag gcgttgatca gatgctgcat ataagcagct gctttttgct tgtactgggt 5280

ctctctggtt agaccagatc tgagcctggg agctctctgg ctaactaggg aacccactgc 5340

ttaagcctca ataaagcttg ccttgagtgc ttcaagtagt gtgtgcccgt ctgttgtgtg 5400

actctggtaa ctagagatcc ctcagaccct tttagtcagt gtggaaaatc tctagca 5457

<210> 12
<211> 8694
<212> DNA

<213>  Artificial

<220>
<223>  Vector for inactivating the huntigtin gene specifically in
      astrocytes

<400>  12
aagcttggcc attgcatacg ttgtatccat atcataatat gtacatttat attggctcat      60

gtccaacatt accgccatgt tgacattgat tattgactag ttattaatag taatcaatta     120

cggggtcatt agttcatagc ccatatatgg agttccgcgt tacataactt acggtaaatg     180

gcccgcctgg ctgaccgccc aacgaccccc gcccattgac gtcaataatg acgtatgttc     240

ccatagtaac gccaataggg actttccatt gacgtcaatg ggtggagtat ttacggtaaa     300

ctgcccactt ggcagtacat caagtgtatc atatgccaag tacgccccct attgacgtca     360

atgacggtaa atggcccgcc tggcattatg cccagtacat gaccttatgg gactttccta     420

cttggcagta catctacgta ttagtcatcg ctattaccat ggtgatgcgg ttttggcagt     480

acatcaatgg gcgtggatag cggtttgact cacggggatt tccaagtctc caccccattg     540

acgtcaatgg gagtttgttt tggcaccaaa atcaacggga ctttccaaaa tgtcgtaaca     600

actccgcccc attgacgcaa atgggcggta ggcgtgtacg gtgggaggtc tatataagca     660

gagctcgttt agtgaaccgg ggtctctctg gttagaccag atctgagcct gggagctctc     720

tggctaacta gggaacccac tgcttaagcc tcaataaagc ttgccttgag tgcttcaagt     780

agtgtgtgcc cgtctgttgt gtgactctgg taactagaga tccctcagac ccttttagtc     840

agtgtggaaa atctctagca gtggcgcccg aacagggacc tgaaagcgaa agggaaacca     900

gagctctctc gacgcaggac tcggcttgct gaagcgcgca cggcaagagg cgaggggcgg     960

cgactggtga gtacgccaaa aattttgact agcggaggct agaaggagag agatgggtgc    1020

gagagcgtca gtattaagcg ggggagaatt agatcgcgat gggaaaaaat tcggttaagg    1080

ccagggggaa agaaaaaata taaattaaaa catatagtat gggcaagcag ggagctagaa    1140

cgattcgcag ttaatcctgg cctgttagaa acatcagaag gctgtagaca aatactggga    1200

cagctacaac catcccttca gacaggatca gaagaactta gatcattata taatacagta    1260

gcaaccctct attgtgtgca tcaaaggata gagataaaag acaccaagga agctttagac    1320

aagatagagg aagagcaaaa caaaagtaag accaccgcac agcaagcggc cgctgatctt    1380

cagacctgga ggaggagata tgagggacaa ttggagaagt gaattatata aatataaagt    1440

agtaaaaatt gaaccattag gagtagcacc caccaaggca aagagaagag tggtgcagag    1500

agaaaaaaga gcagtgggaa taggagcttt gttccttggg ttcttgggag cagcaggaag    1560

cactatgggc gcagcctcaa tgacgctgac ggtacaggcc agacaattat tgtctggtat    1620

agtgcagcag cagaacaatt tgctgagggc tattgaggcg caacagcatc tgttgcaact    1680

cacagtctgg ggcatcaagc agctccaggc aagaatcctg gctgtggaaa gatacctaaa    1740

ggatcaacag ctcctgggga tttggggttg ctctggaaaa ctcatttgca ccactgctgt 1800
gccttggaat gctagttgga gtaataaatc tctggaacag atttggaatc acacgacctg 1860
gatggagtgg gacagagaaa ttaacaatta cacaagctta atacactcct taattgaaga 1920
atcgcaaaac cagcaagaaa agaatgaaca agaattattg gaattagata aatgggcaag 1980
tttgtggaat tggtttaaca taacaaattg gctgtggtat ataaaattat tcataatgat 2040
agtaggaggc ttggtaggtt taagaatagt ttttgctgta ctttctatag tgaatagagt 2100
taggcaggga tattcaccat tatcgtttca gacccacctc ccaaccccga ggggacccga 2160
caggcccgaa ggaatagaag aagaaggtgg agagagagac agagacagat ccattcgatt 2220
agtgaacgga tctcgacggt atcggttatc cagggatcca ccggtatcca tgctagcgga 2280
tccgtcgact agggataaca gggtaattgt ttgaatgagg cttcagtact ttacagaatc 2340
gttgcctgca catcttggaa acacttgctg ggattacttc ttcaggttaa cccaacagaa 2400
ggctcgagaa ggtatattgc tgttgacagt gagcgagcaa gctgaccctg aagttcatct 2460
gtgaagccac agatgggatg aacttcaggg tcagcttgcc tgcctactgc ctcggaattc 2520
aaggggctac tttaggagca attatcttgt ttactaaaac tgaatacctt gctatctctt 2580
tgatacattt ttacaaagct gaattaaaat ggtataaatt aaactcgagg ctagcctgca 2640
gacgcgtgtt aacttttaaa agaaaagggg ggattggggg gtacagtgca ggggaaagaa 2700
tagtagacat aatagcaaca gacatacaaa ctaaagaatt acaaaaacaa attacaaaaa 2760
ttcaaaattt tgtcgacaat caacctctgg attacaaaat ttgtgaaaga ttgactggta 2820
ttcttaacta tgttgctcct tttacgctat gtggatacgc tgctttaatg cctttgtatc 2880
atgctattgc ttcccgtatg gctttcattt tctcctcctt gtataaatcc tggttgctgt 2940
ctctttatga ggagttgtgg cccgttgtca ggcaacgtgg cgtggtgtgc actgtgtttg 3000
ctgacgcaac ccccactggt tggggcattg ccaccacctg tcagctcctt tccgggactt 3060
tcgctttccc cctccctatt gccacggcgg aactcatcgc cgcctgcctt gcccgctgct 3120
ggacaggggc tcggctgttg ggcactgaca attccgtggt gttgtcgggg aagctgacgt 3180
cctttccatg gctgctcgcc tgtgttgcca cctggattct gcgcgggacg tccttctgct 3240
acgtcccttc ggccctcaat ccagcggacc ttccttcccg cggcctgctg ccggctctgc 3300
ggcctcttcc gcgtcttcgc cttcgccctc agacgagtcg gatctccctt tgggccgcct 3360
ccccgcctgg gaattgatcc cgatggctcg ctcaacaaag ggtaaaattc tcttctgggg 3420
ttctggattt tcaccgacag atctctccga aaacctctgt actgttaagg tagcaaagga 3480
ccaaggccaa atgacgctga gatagtctca gcctgggtcc gagggtccag aggtcgctaa 3540
agaacgcgtg ctgcaaaggc gcacagcacc ttcggccccg gccccccagc cgccgctgcc 3600
tcccctcccc cattcccctt tctctttctt tcttctgggt cgctcgcccc accccgcggt 3660
tcctcacggc tcctgggcca atggtctcag ggccccggtg cacagcaact gatgggcacg 3720

gggttccagg cctaggccag ccaatcaggg cgcctggggg cgggcacaag ctgccaataa 3780

aaagtactga gcagcccgca accctccaca gccgagggat ccaagggtgg gcgcgccgac 3840

ccagctttct tgtacaaagt ggtgataatt cctgcagccc gggggatcca ctagttctag 3900

gatccaccat gtctagactg gacaagagca aagtcataaa ctctgctctg gaattactca 3960

atgaagtcgg tatcgaaggc ctgacgacaa ggaaactcgc tcaaaagctg ggagttgagc 4020

agcctaccct gtactggcac gtgaagaaca agcgggccct gctcgatgcc ctggcaatcg 4080

agatgctgga caggcatcat acccacttct gccccctgga aggcgagtca tggcaagact 4140

ttctgcggaa caacgccaag tcattccgct gtgctctcct ctcacatcgc gacggggcta 4200

aagtgcatct cggcacccgc ccaacagaga aacagtacga aaccctggaa aatcagctcg 4260

cgttcctgtg tcagcaaggc ttctccctgg agaacgcact gtacgctctg tccgccgtgg 4320

gccactttac actgggctgc gtattggagg atcaggagca tcaagtagca aaagaggaaa 4380

gagagacacc taccaccgat tctatgcccc cacttctgag acaagcaatt gagctgttcg 4440

accatcaggg agccgaacct gccttccttt tcggcctgga actaatcata tgtggcctgg 4500

agaaacagct aaagtgcgaa agcggcgggc cggccgacgc ccttgacgat tttgacttag 4560

acatgctccc agccgatgcc cttgacgact ttgaccttga tatgctgcct gctgacgctc 4620

ttgacgattt tgaccttgac atgctccccg ggtaactaag taaggatcga tatggcattc 4680

accgcgtgcc ttaattcgaa tggcattcac cgcgtgcctt aaacgcgttg gcattcaccg 4740

cgtgccttaa atgcattggc attcaccgcg tgccttactg cagggtacct taattccgtt 4800

taagaccaat gacttacaag gcagctgtag atcttagcca ctttttaaaa gaaaaggggg 4860

gactggaagg gctaattcac tcccaacgaa gacaagatat gatcccgggc tcgagtttac 4920

cactccctat cagtgataga gaaaagtgaa agtcgagttt accactccct atcagtgata 4980

gagaaaagtg aaagtcgagt ttaccactcc ctatcagtga tagagaaaag tgaaagtcga 5040

gtttaccact ccctatcagt gatagagaaa agtgaaagtc gagtttacca ctccctatca 5100

gtgatagaga aaagtgaaag tcgagtttac cactccctat cagtgataga gaaaagtgaa 5160

agtcgagttt accactccct atcagtgata gagaaaagtg aaagtcgagc tcggtacccg 5220

ggtcgagtag gcgttgatca gatgctgcat ataagcagct gctttttgct tgtactgggt 5280

ctctctggtt agaccagatc tgagcctggg agctctctgg ctaactaggg aacccactgc 5340

ttaagcctca ataaagcttg ccttgagtgc ttcaagtagt gtgtgcccgt ctgttgtgtg 5400

actctggtaa ctagagatcc ctcagaccct tttagtcagt gtggaaaatc tctagcagta 5460

gtagttcatg tcatcttatt attcagtatt tataacttgc aaagaaatga atatcagaga 5520

gtgagaggaa cttgtttatt gcagcttata atggttacaa ataaagcaat agcatcacaa 5580

atttcacaaa taaagcattt ttttcactgc attctagttg tggtttgtcc aaactcatca 5640

atgtatctta tcatgtctgg ctctagctat cccgccccta actccgccca gttccgccca 5700

ttctccgccc catggctgac taattttttt tatttatgca gaggccgagg ccgcctcggc 5760

ctctgagcta ttccagaagt agtgaggagg cttttttgga ggcctaggct tttgcgtcga 5820

gacgtaccca attcgcccta tagtgagtcg tattacgcgc gctcactggc cgtcgtttta 5880

caacgtcgtg actgggaaaa ccctggcgtt acccaactta atcgccttgc agcacatccc 5940

cctttcgcca gctggcgtaa tagcgaagag gcccgcaccg atcgcccttc ccaacagttg 6000

cgcagcctga atggcgaatg gcgcgacgcg ccctgtagcg gcgcattaag cgcggcgggt 6060

gtggtggtta cgcgcagcgt gaccgctaca cttgccagcg ccctagcgcc cgctcctttc 6120

gctttcttcc cttcctttct cgccacgttc gccggctttc cccgtcaagc tctaaatcgg 6180

gggctccctt tagggttccg atttagtgct ttacggcacc tcgaccccaa aaaacttgat 6240

tagggtgatg gttcacgtag tgggccatcg ccctgataga cggtttttcg ccctttgacg 6300

ttggagtcca cgttctttaa tagtggactc ttgttccaaa ctggaacaac actcaaccct 6360

atctcggtct attcttttga tttataaggg attttgccga tttcggccta ttggttaaaa 6420

aatgagctga tttaacaaaa atttaacgcg aattttaaca aaatattaac gtttacaatt 6480

tcccaggtgg cacttttcgg ggaaatgtgc gcggaacccc tatttgttta tttttctaaa 6540

tacattcaaa tatgtatccg ctcatgagac aataaccctg ataaatgctt caataatatt 6600

gaaaaaggaa gagtatgagt attcaacatt tccgtgtcgc ccttattccc ttttttgcgg 6660

cattttgcct tcctgttttt gctcacccag aaacgctggt gaaagtaaaa gatgctgaag 6720

atcagttggg tgcacgagtg ggttacatcg aactggatct caacagcggt aagatccttg 6780

agagttttcg ccccgaagaa cgttttccaa tgatgagcac ttttaaagtt ctgctatgtg 6840

gcgcggtatt atcccgtatt gacgccgggc aagagcaact cggtcgccgc atacactatt 6900

ctcagaatga cttggttgag tactcaccag tcacagaaaa gcatcttacg gatggcatga 6960

cagtaagaga attatgcagt gctgccataa ccatgagtga taacactgcg gccaacttac 7020

ttctgacaac gatcggagga ccgaaggagc taaccgcttt tttgcacaac atgggggatc 7080

atgtaactcg ccttgatcgt tgggaaccgg agctgaatga agccatacca aacgacgagc 7140

gtgacaccac gatgcctgta gcaatggcaa caacgttgcg caaactatta actggcgaac 7200

tacttactct agcttcccgg caacaattaa tagactggat ggaggcggat aaagttgcag 7260

gaccacttct gcgctcggcc cttccggctg gctggtttat tgctgataaa tctggagccg 7320

gtgagcgtgg gtctcgcggt atcattgcag cactggggcc agatggtaag ccctcccgta 7380

tcgtagttat ctacacgacg gggagtcagg caactatgga tgaacgaaat agacagatcg 7440

ctgagatagg tgcctcactg attaagcatt ggtaactgtc agaccaagtt tactcatata 7500

tactttagat tgatttaaaa cttcattttt aatttaaaag gatctaggtg aagatccttt 7560

ttgataatct catgaccaaa atcccttaac gtgagttttc gttccactga gcgtcagacc 7620

ccgtagaaaa gatcaaagga tcttcttgag atcctttttt tctgcgcgta atctgctgct 7680

tgcaaacaaa aaaaccaccg ctaccagcgg tggtttgttt gccggatcaa gagctaccaa 7740

ctctttttcc gaaggtaact ggcttcagca gagcgcagat accaaatact gtccttctag 7800

tgtagccgta gttaggccac cacttcaaga actctgtagc accgcctaca tacctcgctc 7860

tgctaatcct gttaccagtg gctgctgcca gtggcgataa gtcgtgtctt accgggttgg 7920

actcaagacg atagttaccg gataaggcgc agcggtcggg ctgaacgggg ggttcgtgca 7980

cacagcccag cttggagcga acgacctaca ccgaactgag atacctacag cgtgagctat 8040

gagaaagcgc cacgcttccc gaagggagaa aggcggacag gtatccggta agcggcaggg 8100

tcggaacagg agagcgcacg agggagcttc cagggggaaa cgcctggtat ctttatagtc 8160

ctgtcgggtt tcgccacctc tgacttgagc gtcgattttt gtgatgctcg tcaggggggc 8220

ggagcctatg gaaaaacgcc agcaacgcgg cctttttacg gttcctggcc ttttgctggc 8280

cttttgctca catgttcttt cctgcgttat cccctgattc tgtggataac cgtattaccg 8340

cctttgagtg agctgatacc gctcgccgca gccgaacgac cgagcgcagc gagtcagtga 8400

gcgaggaagc ggaagagcgc ccaatacgca aaccgcctct ccccgcgcgt tggccgattc 8460

attaatgcag ctggcacgac aggtttcccg actggaaagc gggcagtgag cgcaacgcaa 8520

ttaatgtgag ttagctcact cattaggcac cccaggcttt acactttatg cttccggctc 8580

gtatgttgtg tggaattgtg agcggataac aatttcacac aggaaacagc tatgaccatg 8640

attacgccaa gcgcgcaatt aaccctcact aaagggaaca aaagctggag ctgc 8694

<210> 13
<211> 5406
<212> DNA
<213> Artificial

<220>
<223> Vector for the inactivation of genes specifically in astrocytes

<400> 13
aagcttggcc attgcatacg ttgtatccat atcataatat gtacatttat attggctcat 60

gtccaacatt accgccatgt tgacattgat tattgactag ttattaatag taatcaatta 120

cggggtcatt agttcatagc ccatatatgg agttccgcgt tacataactt acggtaaatg 180

gcccgcctgg ctgaccgccc aacgaccccc gcccattgac gtcaataatg acgtatgttc 240

ccatagtaac gccaataggg actttccatt gacgtcaatg ggtggagtat ttacggtaaa 300

ctgcccactt ggcagtacat caagtgtatc atatgccaag tacgccccct attgacgtca 360

atgacggtaa atggcccgcc tggcattatg cccagtacat gaccttatgg gactttccta 420

cttggcagta catctacgta ttagtcatcg ctattaccat ggtgatgcgg ttttggcagt 480

acatcaatgg gcgtggatag cggtttgact cacggggatt tccaagtctc caccccattg 540

acgtcaatgg gagtttgttt tggcaccaaa atcaacggga ctttccaaaa tgtcgtaaca 600

actccgcccc attgacgcaa atgggcggta ggcgtgtacg gtgggaggtc tatataagca 660

gagctcgttt agtgaaccgg ggtctctctg gttagaccag atctgagcct gggagctctc 720

tggctaacta gggaacccac tgcttaagcc tcaataaagc ttgccttgag tgcttcaagt 780

agtgtgtgcc cgtctgttgt gtgactctgg taactagaga tccctcagac ccttttagtc 840

agtgtggaaa atctctagca gtggcgcccg aacagggacc tgaaagcgaa agggaaacca 900

gagctctctc gacgcaggac tcggcttgct gaagcgcgca cggcaagagg cgaggggcgg 960

cgactggtga gtacgccaaa aattttgact agcggaggct agaaggagag agatgggtgc 1020

gagagcgtca gtattaagcg ggggagaatt agatcgcgat gggaaaaaat tcggttaagg 1080

ccagggggaa agaaaaaata taaattaaaa catatagtat gggcaagcag ggagctagaa 1140

cgattcgcag ttaatcctgg cctgttagaa acatcagaag gctgtagaca aatactggga 1200

cagctacaac catcccttca gacaggatca gaagaactta gatcattata taatacagta 1260

gcaaccctct attgtgtgca tcaaaggata gagataaaag acaccaagga agctttagac 1320

aagatagagg aagagcaaaa caaaagtaag accaccgcac agcaagcggc cgctgatctt 1380

cagacctgga ggaggagata tgagggacaa ttggagaagt gaattatata aatataaagt 1440

agtaaaaatt gaaccattag gagtagcacc caccaaggca aagagaagag tggtgcagag 1500

agaaaaaaga gcagtgggaa taggagcttt gttccttggg ttcttgggag cagcaggaag 1560

cactatgggc gcagcctcaa tgacgctgac ggtacaggcc agacaattat tgtctggtat 1620

agtgcagcag cagaacaatt tgctgagggc tattgaggcg caacagcatc tgttgcaact 1680

cacagtctgg ggcatcaagc agctccaggc aagaatcctg gctgtggaaa gatacctaaa 1740

ggatcaacag ctcctgggga tttggggttg ctctggaaaa ctcatttgca ccactgctgt 1800

gccttggaat gctagttgga gtaataaatc tctggaacag atttggaatc acacgacctg 1860

gatggagtgg gacagagaaa ttaacaatta cacaagctta atacactcct taattgaaga 1920

atcgcaaaac cagcaagaaa agaatgaaca agaattattg gaattagata aatgggcaag 1980

tttgtggaat tggtttaaca taacaaattg gctgtggtat ataaaattat tcataatgat 2040

agtaggaggc ttggtaggtt taagaatagt ttttgctgta ctttctatag tgaatagagt 2100

taggcaggga tattcaccat tatcgtttca gacccacctc ccaaccccga ggggacccga 2160

caggcccgaa ggaatagaag aagaaggtgg agagagagac agagacagat ccattcgatt 2220

agtgaacgga tctcgacggt atcggttatc cagggatcca ccggtatcca tgctagcgga 2280

tccgtcgact agggataaca gggtaattgt ttgaatgagg cttcagtact ttacagaatc 2340

gttgcctgca catcttggaa acacttgctg ggattacttc ttcaggttaa cccaacagaa 2400

ggctcgagaa ggtatattgc tgttgacagt gagcgatatc gcatgcgcct gcctactgcc 2460

tcggaattca aggggctact ttaggagcaa ttatcttgtt tactaaaact gaataccttg 2520

ctatctcttt gatacatttt tacaaagctg aattaaaatg gtataaatta aactcgaggc 2580

tagcctgcag acgcgtgtta acttttaaaa gaaaaggggg gattgggggg tacagtgcag 2640

gggaaagaat agtagacata atagcaacag acatacaaac taaagaatta caaaaacaaa 2700

ttacaaaaat tcaaaatttt gtcgacaatc aacctctgga ttacaaaatt tgtgaaagat 2760

tgactggtat tcttaactat gttgctcctt ttacgctatg tggatacgct gctttaatgc 2820

ctttgtatca tgctattgct tcccgtatgg ctttcatttt ctcctccttg tataaatcct 2880

ggttgctgtc tctttatgag gagttgtggc ccgttgtcag gcaacgtggc gtggtgtgca 2940

ctgtgtttgc tgacgcaacc cccactggtt ggggcattgc caccacctgt cagctccttt 3000

ccgggacttt cgctttcccc ctccctattg ccacggcgga actcatcgcc gcctgccttg 3060

cccgctgctg gacaggggct cggctgttgg gcactgacaa ttccgtggtg ttgtcgggga 3120

agctgacgtc ctttccatgg ctgctcgcct gtgttgccac ctggattctg cgcgggacgt 3180

ccttctgcta cgtcccttcg gccctcaatc cagcggacct tccttcccgc ggcctgctgc 3240

cggctctgcg gcctcttccg cgtcttcgcc ttcgccctca gacgagtcgg atctcccttt 3300

gggccgcctc cccgcctggg aattgatccc gatggctcgc tcaacaaagg gtaaaattct 3360

cttctggggt tctggatttt caccgacaga tctctccgaa aacctctgta ctgttaaggt 3420

agcaaaggac caaggccaaa tgacgctgag atagtctcag cctgggtccg agggtccaga 3480

ggtcgctaaa gaacgcgtgc tgcaaaggcg cacagcacct tcggccccgg ccccccagcc 3540

gccgctgcct cccctccccc attccccttt ctctttcttt cttctgggtc gctcgcccca 3600

ccccgcggtt cctcacggct cctgggccaa tggtctcagg gccccggtgc acagcaactg 3660

atgggcacgg ggttccaggc ctaggccagc caatcagggc gcctgggggc gggcacaagc 3720

tgccaataaa aagtactgag cagcccgcaa ccctccacag ccgagggatc caagggtggg 3780

cgcgccgacc cagctttctt gtacaaagtg gtgataattc ctgcagcccg ggggatccac 3840

tagttctagg atccaccatg tctagactgg acaagagcaa agtcataaac tctgctctgg 3900

aattactcaa tgaagtcggt atcgaaggcc tgacgacaag gaaactcgct caaaagctgg 3960

gagttgagca gcctaccctg tactggcacg tgaagaacaa gcgggccctg ctcgatgccc 4020

tggcaatcga gatgctggac aggcatcata cccacttctg ccccctggaa ggcgagtcat 4080

ggcaagactt tctgcggaac aacgccaagt cattccgctg tgctctcctc tcacatcgcg 4140

acggggctaa agtgcatctc ggcacccgcc caacagagaa acagtacgaa accctggaaa 4200

atcagctcgc gttcctgtgt cagcaaggct tctccctgga gaacgcactg tacgctctgt 4260

ccgccgtggg ccactttaca ctgggctgcg tattggagga tcaggagcat caagtagcaa 4320

aagaggaaag agagacacct accaccgatt ctatgccccc acttctgaga caagcaattg 4380

agctgttcga ccatcaggga gccgaacctg ccttcctttt cggcctggaa ctaatcatat 4440

gtggcctgga gaaacagcta aagtgcgaaa gcggcgggcc ggccgacgcc cttgacgatt 4500

ttgacttaga catgctccca gccgatgccc ttgacgactt tgaccttgat atgctgcctg 4560

ctgacgctct tgacgatttt gaccttgaca tgctccccgg gtaactaagt aaggatcgat 4620

atggcattca ccgcgtgcct taattcgaat ggcattcacc gcgtgcctta aacgcgttgg 4680

cattcaccgc gtgccttaaa tgcattggca ttcaccgcgt gccttactgc agggtacctt 4740

aattccgttt aagaccaatg acttacaagg cagctgtaga tcttagccac tttttaaaag 4800

aaaagggggg actggaaggg ctaattcact cccaacgaag acaagatatg atcccgggct 4860

cgagtttacc actccctatc agtgatagag aaaagtgaaa gtcgagttta ccactcccta 4920

tcagtgatag agaaaagtga aagtcgagtt taccactccc tatcagtgat agagaaaagt 4980

gaaagtcgag tttaccactc cctatcagtg atagagaaaa gtgaaagtcg agtttaccac 5040

tccctatcag tgatagagaa aagtgaaagt cgagtttacc actccctatc agtgatagag 5100

aaaagtgaaa gtcgagttta ccactcccta tcagtgatag agaaaagtga aagtcgagct 5160

cggtacccgg gtcgagtagg cgttgatcag atgctgcata taagcagctg ctttttgctt 5220

gtactgggtc tctctggtta gaccagatct gagcctggga gctctctggc taactaggga 5280

acccactgct taagcctcaa taaagcttgc cttgagtgct tcaagtagtg tgtgcccgtc 5340

tgttgtgtga ctctggtaac tagagatccc tcagaccctt ttagtcagtg tggaaaatct 5400

ctagca 5406

<210> 14
<211> 21
<212> RNA
<213> Homo sapiens

<400> 14
uaaggcacgc ggugaaugcc a 21